(19) Europäisches Patentamt
European Patent Office
Office européen des brevets

(11) **EP 4 678 168 A1**

(12) **EUROPEAN PATENT APPLICATION**

(43) Date of publication:
**14.01.2026  Bulletin 2026/03**

(21) Application number: **25188288.2**

(22) Date of filing: **08.07.2025**

(51) International Patent Classification (IPC):
**A61K 9/20** *(2006.01)*    **A61K 9/00** *(2006.01)*
**A61K 31/593** *(2006.01)*    **A61K 31/714** *(2006.01)*

(52) Cooperative Patent Classification (CPC):
**A61K 9/0056; A61K 9/2009; A61K 9/2013;
A61K 9/2018; A61K 9/2054; A61K 31/593;
A61K 31/714**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority:  **09.07.2024  IT 202400015844**

(71) Applicant: **Unifarco S.p.A.**
**32035 Santa Giustina (BL) (IT)**

(72) Inventors:
• **Baratto, Giovanni**
**32035 Santa Giustina BL (IT)**
• **Baracchini, Silvia**
**32035 Santa Giustina BL (IT)**
• **Tafuro, Giovanni**
**32035 Santa Giustina BL (IT)**
• **Casanova, Elena**
**32035 Santa Giustina BL (IT)**
• **Faggian, Marta**
**32035 Santa Giustina BL (IT)**

(74) Representative: **Perani & Partners S.p.A.**
**Corso Europa, 15**
**20122 Milano (IT)**

(54) **TABLETS OBTAINED BY POWDER COMPRESSION COMPRISING AS DILUTING AND LUBRICATING AGENTS COMPOUNDS OF NATURAL ORIGIN**

(57)  Tablets obtained by compression of powder mixtures comprising:
a) at least one active ingredient,
b) at least one diluent;
c) at least one lubricating agent
d) at least one excipient selected from at least one of the following classes: acidifier, binder, disaggregating agent, anti-caking agent, sweetener, stabilizer

wherein:
i) the diluent b) is a mixture of at least one conventional diluent and fructooligosaccharides (FOS),
ii) the lubricating agent is a mono- and/or diglyceride of a fatty acid;
iii) the FOS/conventional diluent ratio is between 0.5 and 3.

EP 4 678 168 A1

**Description**

STATE OF THE ART

The present invention relates to tablets obtained by compression of powders comprising compounds of natural origin as diluents and lubricants.

STATE OF THE ART

In the current context of the pharmaceutical and nutraceutical sector for the development of products in solid forms, the study of the characteristics of the excipients that carry the active ingredient has become increasingly crucial. Properties such as particle shape, size, density and rheological behaviour are fundamental to analyse and understand the behaviour of each powder and thus optimise the production process and the quality of the finished product.
In relation to the difficulties in the supply of raw materials linked to the international situation of recent years and the attention by Authorities and consumers that has often led to the prohibition of the use of some ingredients, such as titanium dioxide, within foods and food supplements, a rapid analysis and identification of possible alternatives is required.
Today, also to meet the needs of an increasingly saturated and competitive market, some companies offer "alternative" food-grade excipients to the more conventional ones typical of the pharmaceutical industry, developing substances and preparations that can appeal to consumers both in terms of naturalness and functional effectiveness. Among these, some ingredients of natural origin have been developed as substitutes for synthetic disaggregating agents and binders.
In this context, rheology qualifies as a method of great interest for the possibility of measuring properties and variables such as flowability, flow, consolidation, density, compressibility, segregation and/or friction.
Food supplements are defined by the legislation (Directive 2002/46/EC, implemented by Legislative Decree no. 169 of 21 May 2004) as: "foodstuffs the purpose of which is to supplement the normal diet and which are concentrated sources of nutrients, such as vitamins and minerals, or other substances with a nutritional or physiological effect, in particular, but not exclusively, amino acids, essential fatty acids, fibre and various plants and herbal extracts, both single- and multi-compound, in pre-dosed forms".
Food supplements are usually produced in dosage forms such as capsules, tablets, sachets, vials and can contribute to daily well-being by providing nutrients or other substances with a nutritional and/or physiological effect.
Food supplements contain a variety of elements whose quantity must be carefully monitored and comply with strict regulations. In addition to the main ingredients, food supplements also include excipients, which are inert substances, added for the purpose of protecting, preserving and conveying the active ingredient["What are food supplements? Are they really needed? | Mario Negri." Accessed: Apr. 03, 2024. [Online]. Available: https://www. marionegri.it/magazine/integratori-alimentari#].
Nowadays, the tablet is considered the simplest and most convenient pharmaceutical form for the oral administration of drugs and food supplements.
Within the Official Italian Pharmacopoeia ed.XII there is a subdivision of tablets into different types [P. Colombo and S. Nicoli, "Compresse," in Principi di Tecnologia Farmaceutica, Casa Editrice Ambrosiana, Ed., Milan, 2004, pp. 251-268.]:

- uncoated, monolayer or multilayer tablets, the latter being mainly used for substances incompatible with each other;
- coated tablets, coated with certain substances for the purpose of masking unpleasant tastes and odours, protecting against moisture and light and finally improving performance;
- effervescent tablets, dissolved in water before oral administration;
- soluble tablets, can be coated and must be dissolved or dispersed in water prior to oral administration;
- orodispersible tablets, dissolved in the mouth before being swallowed;
- modified-release tablets, formulated with technologies or excipients such as to allow the control of the release of active ingredient after administration;
- gastro-resistant tablets, coated with a layer resistant to the acidic pH of the stomach allowing the release of the active ingredient at pH above 5, i.e. in the intestine;
- tablets to be used in the buccal cavity.

Tablets are made from granulations or mixtures of powders, made using the following general techniques:

- wet granulation, carried out by distributing a liquid in the powder, which leads to the obtaining of interactions between the particles and a liquid phase that incorporates the solid one;
- dry granulation, carried out by mechanically compacting the powder thereby obtaining granules of the desired size, crushing the masses thus obtained;

- direct compression.

Among these, the most used technique is wet granulation, but the manufacturing of tablets for direct compression is constantly increasing, as it offers economic advantages thanks to the elimination of the wet granulation and drying phases [G. K. Bolhuis and Z. T. Chowhan, "Material for Direct Compaction," in Pharmaceutical Compaction Powder Technology, G. Alderborn and C. Nystrom, Eds., Uppsala, Sweden, 1995, p. 419.]. Tablets are prepared by uniformly compressing volumes of particles or granules. Their manufacturing requires the use of complex equipment called tablet presses which can be divided into two categories: alternative tablet presses and rotary tablet presses. The main distinction between these two groups is highlighted by the different operating cycles used during the compression process, which affects both the characteristics of the tablets and the production speed.

The compression process consists of pressing into a mould, known as a die, the powder or granulates. This is done by the use of so called punches, which exert pressure on the powder within the die. Both the punches and the die are made of hardened steel, resistant to high pressures.

The configuration of the die defines the shape of the tablet, while the thickness of the tablet is determined by the distance between the inner surfaces of the punches. In addition, the internal geometry of the punches affects the characteristics of the tablet faces, determining whether they will be flat or concave.

Regardless of the type of tablet press used, the process can be divided into three phases: filling the die, compressing the material, and finally ejecting the tablet.

The main difference between the alternative and the rotary tablet press lies in the mechanism of action, i.e. in the movement of the punches during the compression process: an up-and-down alternative movement for the alternative tablet presses and a rotary movement for the rotary tablet presses. The choice between these two types depends on specific production needs, including speed, production capacity, and cost.

The mass to be compressed must have specific requirements for proper compression: uniformity of distribution in the die, flowability of the particles inside the compressor, aggregatability and poor adhesion.

In turn, the tablets also require precise quality requirements and compliance with the following criteria[9]:

- they must allow the release of the active ingredient and subsequent absorption;
- they must ensure accuracy and uniformity of dosage of the active ingredient within the tablet;
- they must guarantee chemical and physical stability;
- they must have a reassuring size, colour, odour and taste for the consumer.

These latter properties are very important, as the first evaluation that is made by the patient/consumer concerns the aesthetic appearance of the tablet. It is essential that the tablet is free of aesthetic defects, has a shape that is easy to swallow, and features a different colour for each type of drug/food supplement, so that one or more therapies can be followed without making mistakes.

The tablets must contain precise amounts of active ingredient, be sufficiently resistant to any subsequent handling, and, in addition, they must allow for easy disaggregation upon contact with water or dissolve readily.

The main tests performed on tablets consist of carrying out various assays listed in the Italian Official Pharmacopoeia, 12th Edition, including:

- the mass uniformity assay which consists in "individually weighing twenty units randomly selected from the same batch or, for single-dose preparations packaged individually, the contents of twenty units, and determining the average mass. (...)"
- the content uniformity assay which "is based on the determination of the individual contents of the active ingredient in a number of single-dose units, to verify whether they are included within the limits established in relation to the average content of the sample. (...) Randomly taking ten dosage units and determining, using a suitable analytical method, the individual contents of active ingredient in each of them". The assay is satisfied if all tablets fall within the range 85-115% of the average content.
- the assay for the determination of the friability of the tablets consists of "accurately weighing the sample of tablets and placing it in the drum. Rotating the drum one hundred times and removing the tablets. (...) For most products, a mass loss of 1 percent, obtained as a result of a single assay or the average of three assays, is considered acceptable".

- the disaggregation assay is used to determine "whether the tablets or capsules, when placed in a liquid medium under the indicated experimental conditions, disaggregate within the prescribed time".
- the dissolution assay for solid pharmaceutical forms is very important with regard to the bioavailability of the drug, "it is used to determine the compliance of solid oral dosage forms with dissolution requirements".

Due to possible problems during the manufacturing phase or due to an inadequate formulation, the tablets may exhibit some defects, among them the main ones concern a non-constant weight, poor mechanical strength, difficult disintegrability, decapping and delamination. This may be due to an incorrect filling of the die due to the quality of the granulate or the machine itself.

The phenomenon of decapping causes the detachment of the lower or upper part of the tablet, while delamination consists of splitting the tablet into two or more parts.

The causes of these two phenomena are multiple, but most of the time they concern an incorrect removal of the air during the compression phase, which will then be released later, thus giving rise to decapping or delamination. Another cause of these phenomena is due to a pressure exerted too quickly by the compressor that does not allow air to escape.

Other disadvantages of tablets are related to storage problems, irregular absorption or difficulty in swallowing.

In this perspective, the choice of formulation and excipients plays a very important role in avoiding these defects in the compression phases or in the final product.

The term "excipient" comes from the Latin *excipere* and means to receive. The excipients have the task of receiving the active ingredient, transporting it and keeping it in good condition and, to do this, they must be inert, neutral [M.-C. Martini, "Eccipienti cosmetici o ingredienti tecnologici", EMC - Cosmetologia Medica e Medicina degli Inestetismi Cutanei, vol. 19, no. 1, pp. 1-9, Mar. 2022, doi: 10.1016/S1776-0313(21)46007-7].

In the Official Italian Pharmacopoeia ed.XII we can find the following definition: "any component, other than the active ingredient, present in a pharmaceutical preparation or used for its manufacturing".

They play an important role in the formulation of tablets and other pharmaceutical forms and can also be used to improve the bioavailability of drugs [S. Chaudhari P and P. Patil S, "Pharmaceutical Excipients: A review," International Journal of Advances in Pharmacy, Biology and Chemistry, 2012].

These substances, for the safety and efficacy of the drug, require: stability throughout the formulation process until the consumption of the drug by the patient, protection and bioavailability.

Today we have numerous plant-derived pharmaceutical excipients, such as starch, agar, alginates, guar gum, xanthan gum, gelatin, and cellulose. These natural excipients find application in the pharmaceutical industry as binding agents, disaggregating agents, lubricants, protectives, diluents, thickeners, gellants, stabilizers and coating materials. Their advantages certainly include low cost, natural origin, no side effects, biocompatibility, environmentally friendly processing, local availability and better consumer tolerance. There are also chemically synthesized excipients or semi-synthetic excipients, obtained from naturally occurring materials. We can find simple, well-characterized organic or inorganic molecules and materials that are highly complex and difficult to fully characterize [T. Kumar, S. Kumar Gupta, K. Prajapati, D. K. Tripathi, V. Sharma, and P. Jain, "Natural Excipients: A Review," Asian Journal of Pharmacy and Life Science, vol. 2, no. 1, Accessed: Apr. 07, 2024. [Online]. Available: www.ajpls.com], [C. G. Abrantes, D. Duarte, and C. P. Reis, "An Overview of Pharmaceutical Excipients: Safe or Not Safe?" J Pharm Sci, vol. 105, no. 7, pp. 2019-2026, Jul. 2016, doi: 10.1016/J.XPHS.2016.03.019].

Excipients are classified by the role they play during the drug manufacturing process and in the process of releasing the active ingredient. Let's see in detail the different classes of excipients that are most used in numerous industrial sectors according to their function[E. Menegatti, A. Scatturin, F. Bortolotti, and A. Dalpiaz, "Componenti delle preparazioni farmaceutiche - Eccipienti," in Principi di Tecnologia Farmaceutica, Casa Editrice Ambrosiana, Ed., Milano, 2004, pp. 155-158*]:*

- **Diluents,** are used when the amount of the active ingredient is insufficient to reach the desired volume of the tablet, thus helping to reach the right weight. These substances can be soluble or insoluble, organic or inorganic and among the most used are sugars (mannitol, sucrose, lactose), starch and different types of cellulose.
- **Binders,** are employed to promote cohesion of the powder particles, as usually the components of a formulation are not naturally cohesive enough to form a tablet, even under high pressure. The percentages and doses of these ingredients must be carefully adjusted to allow, after intake, the disaggregation process. Some examples of binders are gums (xanthan, arabic), gelatin, some cellulose derivatives and sucrose.
- **Lubricants,** prevent friction and wear by interposing between two moving surfaces. These excipients can be divided into glidants, i.e. ingredients that reduce interparticle friction by trying to increase flowability of the material to be compressed, and as non-stick agents, used during the ejection phase of the compression process to prevent the tablet from adhering to the die walls. The most commonly used are magnesium stearate, silica and talc.
- **Disaggregating agents,** whose main function is to allow the process of disaggregation in contact with water through the weakening of the binder and the cohesive forces between the particles. They are usually insoluble substances that swell upon contact with water leading to breakage of the tablet. Examples include starch, cellulose, and some synthetic polymers.
- **Surfactants,** their use is very useful in the case of highly hydrophobic substances because, thanks to their wetting action, they are able to promote contact with water allowing the tablet to disaggregate.
- **Colourants** are excipients added essentially to distinguish one product from another, to control the various stages of the manufacturing process and for aesthetic reasons. The limit of use of these substances is 0.03% (w/w).

- **Flavourings and sweeteners,** mostly used in chewable or orodispersible tablet formulations to make the tablet pleasant. In this case, the maximum concentration to be used is 0.5% (w/w).
- **Adsorbents,** used in the case of active ingredients such as oils, plant extracts or essences inside the tablet. They are able to maintain a dry appearance by being able to retain high amounts of liquid. Among the most used adsorbents is silica gel, capable of adsorbing up to 50% of its weight in water.
- **Humectants,** essential to maintain a constant level of humidity and to reduce the process of water evaporation.
- **Antimicrobials,** prevent microbial growth in pharmaceutical preparations (benzalkonium chloride, sorbic acid).
- **Antioxidants,** limit the oxidative degradation of the active ingredient (ascorbic acid).
- **Stabilizers,** "stabilizers" are substances that make it possible to maintain the physico-chemical state of foodstuffs; stabilizers include substances that make it possible to maintain a homogeneous dispersion of two or more immiscible substances in a foodstuffs, substances that stabilize, retain or intensify the coloration of foodstuffs and substances that increase the ability of food to form bonds, including the formation of crosslinks between proteins that allow the bonding of particles for the formation of reconstituted food. (Annex I, REGULATION (EC) No. 1333/2008 OF THE EUROPEAN PARLIAMENT AND OF THE COUNCIL of 16 December 2008 on food additives)

By the term powder is meant a group, generally heterogeneous, of solid, dry and free particles. Powders represent as such a pharmaceutical form or are used for the preparation of other solid pharmaceutical forms such as granules, tablets, capsules or dispersed pharmaceutical forms such as suspensions or colloidal dispersions [R. Bettini, S. Cagnani, and M. Artusi, "Polveri" Casa Editrice Ambrosiana, Ed., Milan, 2004, pp. 231-247].

The granular materials represent complex systems whose operation is still poorly understood. This is because the characteristics of a granular solid are influenced by a number of factors, generally divided into three main categories: intrinsic physical properties, process conditions and external environmental conditions. These factors include size, shape, roughness, porosity, mass density, and interactions between particles, along with variables such as stress, strain rate, storage time, temperature, and humidity. In addition, the same powder can assume gas-, liquid-, or solid-like behaviours at different stress levels [G. Alderbor, R. Rowe C., R. Roberts J., M. Wikberg, and W. Duncun-Hewitt C., "Material properties of importance for powder volume reduction and compact strenght," in Pharmaceutical Powder Compaction Technology, I. Marcel Dekker, Ed., New York, 1995, pp. 245-375].

The powders, nowadays, are widely used in the pharmaceutical, cosmetic, nutraceutical and food fields.

The properties of the powders can be classified as:

o Basic properties
o Derived Properties

The fundamental properties are characterized by size, shape and surface area, intrinsic density of each individual particle. The derived properties, or bulk properties, such as density, porosity and flowability, concern the particles considered as a whole.

They are both very important as they influence the practical aspects of pharmaceutical formulations, such as e.g. rheological properties, packing properties, dissolution or sedimentation rate.

The fundamental properties of powders include their size, shape and surface area.

In the process of handling and processing powders, the size and distribution of the particles play a crucial role in determining their characteristics. In fact, many properties derive from them, including, for example, density, porosity, settling and flowability. The micrometer ($\mu$m) is the unit of measurement that describes the size of the particles, which can be divided into four classes of fineness according to the Official Pharmacopoeia:

- \> 355 $\mu$m: coarse powders;
- 355 - 180 $\mu$m: moderately fine powders;
- 125 - 180 $\mu$m: fine powders;
- $\leq$ 125 $\mu$m: very fine powders;

There are also micronized powders that have a size of less than 10 $\mu$m.

In many industrial environments, an attempt is made to characterize particle sizes using a single value. As a rule, particles have an irregular shape different from the spherical shape and this makes it difficult to identify a single value to accurately describe their dimensions [M. Rhodes, "Particle Size Analysis," in Introduction to Particle Technology, M. Rhodes, Ed., Monash University, Australia, 2008, pp. 1-25.].

There are several methods to calculate the diameter of an irregular particle, but the most widely used is certainly the calculation of the equivalent spherical diameter.

Being able to assign to a particle with irregular dimensions the dimension of an equivalent sphere is a very important data to go on to define the diameter, volume and surface.

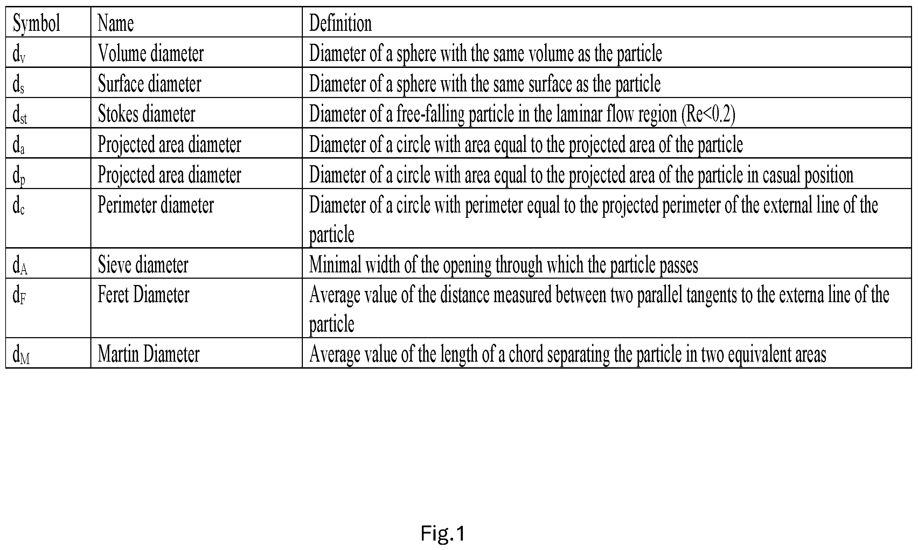

In Figure 1 the most relevant equivalent spherical diameters in pharmaceutical technology are shown. Next to each of them, the property considered equivalent is indicated.

There are other methods for determining the size of a powder:

- SIEVING is a simple and economical method of dimensional analysis, suitable for particles larger than 45 mm. It consists of using sieves with a metal mesh of different sizes, stacked one on top of the other (figure 1.10). They are moved by a vibration that allows the powder to pass from one sieve to another, determining its size.
- LASER DIFRATOMETRY, widely used to determine particle distribution in the pharmaceutical field. Low-intensity lasers are used, for example those with He-Ne gas with a wavelength of 630 nm. "The technique is based on the fact that light is refracted by a particle with an angle that is inversely proportional to its volume. A laser light detector uses the intensity of the radiation refracted by the particles. These are passed through the laser beam by free fall and pushed by a jet of compressed air.
- MICROSCOPIC TECHNIQUES, the optical microscope is suitable for measuring particle sizes up to 5 mm. For smaller particles, diffraction causes blurring of the edges, generating an apparent size. For the size of less than 5 mm, it is preferred to use the electron microscope. Both, when combined with an image analysis system, allow for easy numerical distributions of particle size and shape.
- COULTER COUNTER, is a conductometric method where the test sample is suspended within an electrolyte solution, e.g. NaCl, and this suspension is passed through a small orifice within a counting chamber. When the particles pass through the orifice, each of them causes a momentary decrease in electrical resistance between two electrodes placed on the sides of the orifice. This change in resistance is proportional to the particle volume [J. Hurley, "SIZING PARTICLES WITH A COULTER COUNTER", doi: 10.1016/S0006-3495(70)86286-5].

It is also important to know the value of the frequency with which the value of the particle size is repeated in a polydisperse group of particles.

This can be achieved by entering the values of the various measurements in a dimensional range defined by a lower limit and an upper limit, within which the numbers of particles with a value within that certain range will be assigned. This will result in a histogram characterised by a Gaussian curve from which, one will be able to read and interpret data that will allow one to understand the frequency of each individual particle at a given diameter within a heterogeneous system.

The shape of the particles may affect other properties such as particle-particle interaction, flow and packing.

As with the size, it is also useful to use the comparison with a sphere for the shape. To achieve this goal, parameters called shape factors are used, which allow the volume and surface area of a non-spherical particle to be calculated starting from a known equivalent diameter of the same

By referring to the known value of a sphere, we can evaluate how far the shape of a particle deviates from the sphere. The ratio between the shape factors, which concern the surface and the volume of an irregular particle, offers a numerical indication of its "non-sphericity": the greater the particle's symmetry, the greater the value of this ratio "The specific surface area of a powder represents the area of the powder itself per unit of volume or per unit of weight".

The methods used to determine the specific surface area of a powder are essentially two:

- The adsorption method, known today as BET technique. This technique consists in the adsorption of a gas or a liquid on the powder to be examined and the amount of the adsorbed material is proportional to the surface area of the powder.
- The air permeability method allows the surface area to be determined by considering the ease with which a liquid or gas passes through the powder bed. In

fact, the permeation resistance is given by the surface area of the powder.

Instead, the properties derived from the powders are porosity, density and flowability.

Regarding porosity, a powder column represents a heterogeneous system composed of solid particles and air. Air can be present both between the particles (interparticle spaces) and within them (interparticle spaces). The physical characteristics of a powder column are different from those of a solid, since the powder can flow and manifest rheological properties similar to those of liquids and, at the same time, the powders can undergo permanent deformations (plastic flow), reversible deformations (elasticity) and fractures, typical phenomena of solids.

Porosity is defined as the ratio of the volume of the voids to the apparent volume of the powder. However, three types of volume must be considered:

- The volume of the entire particle without considering the intra and interparticle spaces (true volume).
- The volume without considering the open pores, but only the closed ones (apparent volume).
- The volume of the particle and the open and closed pores (envelope volume).

Once these types of volumes are defined, we can say that there are essentially two main porosities: **intraparticulate porosity** (considers the open and closed pores of the particle) and **interparticulate porosity** (considers the gaps between the various particles). Density is expressed as weight per unit volume. The determination of the weight of a powder is easily determined, while it is more complex to accurately measure its volume. Depending on the type of volume used, three different densities will be obtained:

1) <u>True density</u>, represents the density of the solid and is calculated from the ratio of weight to the true volume of the particle, thus excluding all intra and interparticle spaces
2) <u>Granular density</u>, uses the envelope volume of the particle including open and closed pores.
3) <u>Apparent density</u> (or bulk density), calculated considering the true volume of the particle and the intra and interparticle spaces.

The apparent volume assay is performed to determine the apparent volumes before and after packing, the ability to pack, and the apparent densities of solids (e.g., powders, granules).
Despite the difficulty in characterizing the flowability of a powder, there are several methodologies to evaluate its flow. These techniques can be divided into two major categories: direct methods, which directly observe the behaviour of granular material during flow in its consolidated state, and indirect methods, which determine flow properties related to fluidity in its uncompacted state [A. Santomaso, P. Lazzaro, and P. Canu, "Powder flowability and density ratios: the impact of granules packing," Chem Eng Sci, vol. 58, no. 13, pp. 2857-2874, Jul. 2003, doi: 10.1016/S0009-2509(03)00137-4].
The angle of repose, the compressibility index or Hausner index and the flow rate through an orifice are indirect methods reported in the Italian Official Pharmacopoeia, while the use of a shear or flow cell is a direct method.
The angle of repose is a practice commonly employed to evaluate the flow characteristics of powders and solid materials. This is the angle formed by a conical-shaped pile when it is overturned on a flat surface. The angle of repose provides a reliable indication of the interparticle friction or resistance to movement linked to the interaction between particles.
The application of this method consists of placing a horizontal surface on a support and pouring the material over it through a funnel, until a maximum height is reached [R. G. Holdich, "Powder flow and storage," in Fundamentals of Particle Technology, Midland Information Technology and Publishing, Ed., Leicestershire, U.K.].
The angle of repose can be determined by the following geometric relationship:

$$\alpha_r = \tan^{-1}\left(\frac{2h}{D_a - d}\right),$$

where $D_a$ represents the diameter of the conical base of the material, while $d$ is the diameter of the mouth of the funnel.
The measurement methods of this angle are essentially two and can be classified in relation to two experimental variables:

- "the height of the funnel, through which the powder passes, can be fixed with respect to the base, or it can vary as the pile forms".
- "The base on which the powder pile forms may have a fixed diameter, or the diameter of the powder cone may be allowed to vary as the pile forms".

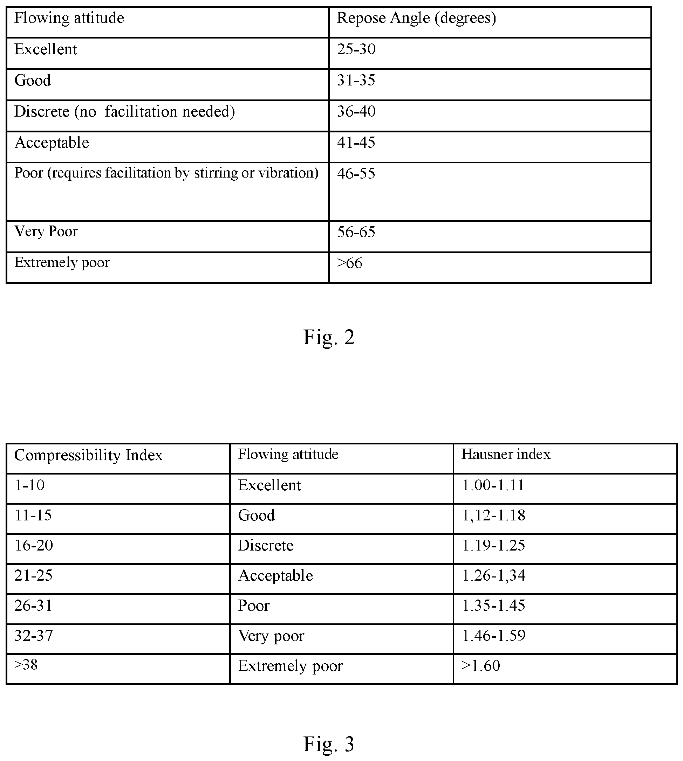

This measurement method is quick, easily repeatable, and simple to perform. However, its reproducibility becomes more complicated with cohesive materials, as angles of repose with steeper and irregular piles can be observed due to increased friction and interactions between particles. Another limitation of this technique concerns the large amount of material required to conduct the tests.
Significant values occur when the angle does not exceed 50 degrees, since poorly flowing and highly cohesive granular materials can subsequently cause problems in the manufacturing processes (Figure 2 shows the flowability as a function of the angle of repose.)
The compressibility index and the Hausner index are indicators proposed in the literature that are used to define the flowability of a powder; in addition, they both measure the propensity of a powder to be compressed and allow the extent of interparticle interactions to be evaluated.
The compressibility index, also called *Carr Index,* evaluates how the density of a material varies under the effect of applied normal forces and, therefore, measures the tendency of a powder to compact. The Hausner index, on the other hand, indicates the cohesion of a loose solid material. Both are qualitatively equivalent methods, since they are based on the same variables.
The parameters that are considered for the calculation of these two factors are the tapped density $\rho_t$ calculated considering the volume value after the sample has been compacted ($V_f$) and the bulk density $\rho_b$, calculated considering the volume of the particle including the inter- and intra-particle spaces ($V_0$).

The equations used for the calculation of the compressibility index (*IC*) and the Hausner index (HR) are as follows:

$$IC[\%] = 100 \; \frac{\rho_t - \rho_b}{\rho_t} = \; 100 \; \frac{V_0 - V_f}{V_0} \, ,$$

$$HR = \frac{\rho_t}{\rho_b} = \frac{V_0}{V_f}$$

Considering the variations present between the densities before and after the tapping process, which is obtained by mechanically compacting a sample of powder contained in a cylinder, it is possible to obtain a significant indication on powder flowability and on its degree of compacting. In materials that tend to minimise the voids once poured, this variation is minimal. In contrast, in the more cohesive powders, a more noticeable difference between the two densities is observed. In addition, powder flowability increases with increasing particle size and there appears to be a size critical value above which powder flow does not improve [E. C. Abdullah and D. Geldart, "The use of bulk density measurements as flowability indicators," Powder Technol, vol. 102, no. 2, pp. 151-165, Mar. 1999, doi: 10.1016/S0032-5910(98)00208-3].

In figure 3 a scale of flowability is shown, listing the values that the two indexes may exhibit in relation to the powder's flowability characteristics.

Since the Hausner index and Carr index are not intrinsic characteristics of powders, there is an ongoing discussion about the reliability of the two indicators in assessing powder flow. [M. A. Kaleem, M. Z. Alam, M. Khan, S. H. I. Jaffery, and B. Rashid, "An experimental investigation on accuracy of Hausner Ratio and Carr Index of powders in additive manufacturing processes," Metal Powder Report, vol. 76, pp. S50-S54, Dec. 2021, doi: 10.1016/J.MPRP.2020.06.061].

For example, in some cases discordant results could be obtained and this could result from a different filling of the sample or a difference in the compaction of the material; such divergences are usually influenced by the individual operator performing the test.

The flow rate through an orifice falls into the group of indirect methods and is one of the most widely used for determining the flowability of a powder. The instruments with which to characterize the powder takes into account all the physical characteristics related to flowability, such as size, shape, surface, porosity, electrostatic charge. With this technique, the amount of powder that passes through an orifice in a given period of time is observed.

The flow rate through an orifice is strictly dependent on the method employed. Several parameters can be found in the literature that can influence the measurements:

- the diameter and shape of the orifice,
- the nature of the container material (metal, glass, plastic)
- the diameter and height of the powder bed.

The instruments used consists of a funnel and three nozzles of different diameters, 25 mm, 15 mm and 10 mm, with quick change in stainless steel, designed to simulate the flow of powder in a hopper or in an industrial silo.

One of the disadvantages of this method is that some powders with cohesive behaviour tend to form arcs, interrupting continuous flow.

Furthermore, there is no definite relationship between the flow rate and the properties of the powder flow, which means that it is not possible to directly compare this method with the previously mentioned flow indicators.

The shear cell technique, initially developed by A.W. Jenike is one of the most used methodologies to evaluate rheology, i.e. powder flowability. This method is used to evaluate the flow properties of the powders and to understand how the state of consolidation affects these properties. In addition, it has been documented and standardized internationally [Y. Wang, S. Koynov, B. J. Glasser, and F. J. Muzzio, "A method to analyze shear cell data of powders measured under different initial consolidation stresses," Powder Technol, vol. 294, pp. 105-112, Jun. 2016, doi: 10.1016/J.POWTEC.2016.02.027].

It is classified within the category of "direct methods" since, through this technique, it is possible to directly evaluate the behaviour of the material during movement, taking into account the initial stress applied.

Among the various types of shear cells, the best known are Jenike's and the annular one. In the first case, a translational shear test is performed; however, its execution requires long times, high amounts of material and, in addition, the results may vary according to the operator's manual skills.

In the translational shear cell a normal force is applied to the lid, the upper ring is moved horizontally keeping the lower one still, causing a deformation of the solid. Normal stress ($\sigma$) and shear stress ($\tau$) are determined by dividing the normal force and shear force by the cross-sectional area A of the shear cell [D. Schulze, "Flow Properties of Powders and Bulk Solids".].

The Schulze annular shear cell, on the other hand, widely used in the pharmaceutical industry, consists of a rotational shear cell. It allows multiple shear tests to be performed on a powder sample to measure different yield loci, which represent the powder's yield under a given normal stress at various stress levels. Thus, it provides a relatively rapid evaluation of the relationship between the stress and flowability of a powder using the same material[T. Swize, F. Osei-Yeboah, M. L. Peterson, and P. Boulas, "Impact of Shear History on Powder Flow Characterization Using a Ring Shear Tester," J Pharm Sci, vol. 108, no. 1, pp. 750-754, Jan. 2019, doi: 10.1016/J.XPHS.2018.07.003].

In this case, the lower ring of the shear cell contains the sample with an upper lid fixed to a vertical axis. A normal force (FN) is applied on the traverse in the rotation axis of the shear cell and transmitted to the sample. In this way, a normal stress ($\sigma$) is applied. There is also a counterbalancing force, FA, which acts at the centre of the vertical axis and counteracts the forces of gravity of the lid, hook and transverse axis [A. Santomaso, P. Lazzaro, and P. Canu, "Powder flowability and density ratios: the impact of granules packing," Chem Eng Sci, vol. 58, no. 13, pp. 2857-2874, Jul. 2003, doi: 10.1016/S0009-2509(03)00137-4.]. After the shear stress is applied, the lid and the ring, which are both annular, rotate creating a shear deformation inside the solid bulk [D. Schulze, "Flow Properties of Powders and Bulk Solids".].

Some advantages of this technique are a high degree of control and the possibility of obtaining additional useful parameters, such as flow factor, internal and wall friction, cohesion and shear strength with the use of a single sample.

However, these types of shear cells also have some limits, for example the amount of sample to be used is usually between 200 g and 300 g, which, in the case of analyses in pharmaceutical companies with the use of very expensive experimental materials, is not advantageous.

As for the process with which the characterization of the powders takes place with respect to their flow by means of a shear cell, the same includes the steps of:

- initially subjecting the sample to a constant normal load (said pre-consolidation stress or pre-shear $\sigma_{pre}$) until the shear stress value reaches a plateau ($\tau_{pre}$), i.e. a steady-state situation where density and shear rate remain constant (steady-state).
- Subjecting it to a shear stress given by a normal stress lower than the initial one until a maximum shear stress at which the material yields (yield strength or incipient flow) representative of the strength of the sample under those certain conditions is reached.
- Repeating this procedure several times at decreasing normal load values between 20% and 80% of the initial pre-shear value, obtaining different yield strengths.

Each pair of points (normal stress $\sigma$; shear stress $\tau$) is graphed to form the internal yield locus of the points of the material in the state of consolidation, which for an ideal material corresponds to a straight line.

The line follows Coulomb's equation:

$$\tau = \mu\sigma + C,$$

where $\tau$ is the observed shear stress, $\sigma$ the normal consolidation stress, $\mu$ represents the coefficient of friction and C is the cohesion to the shear stress.

At this point, it is possible to represent two Mohr circles tangent to the yield locus curve obtained, in which the smallest passes through the origin of the axes, while the largest through ($\sigma_{pre}$; $\tau_{pre}$). Taking the intersection of these circles with the axis of the abscissas, two key values of normal stress are found respectively: the unconfined yield stress $f_c$ and the maximum principal stress $\sigma 1$ or $\sigma x$.

The first is the force required for the powder to begin to yield when it is not confined within a surface, while the second term represents the greatest principal stress applied to the powder within a container.

The ratio between $\sigma_x$ and $f_c$ defines the flow function (PFF).

According to the different values that this function can assume, it is possible to classify and characterize the powder flowability in five categories:

- *free flowing*
- *easy flowing*
- *choesive*
- *very choesive*
- *not flowing*

The study of the characteristics of powders according to the type of excipients and their particle distribution can benefit, in addition to the classic techniques for the study of flowability and density properties, also of the measurement of rheological behaviour, for the purpose of comparing excipients with each other, identifying any synergistic associations, optimizing formulas avoiding inconveniences during the delicate phases of production, quality control and packaging and reformulating products on the market that have greater appeal for the consumer.

The work was divided into two phases. The first phase involved the characterization of individual ingredients of natural origin in comparison with conventional excipients, specifically evaluating their flowability by flowability tester and shear cell, their density by tapped density tester and their behaviour when varying the temperature by differential scanning calorimetry (DSC).

After analysing the rheological and flowability characteristics of multiple ingredients of natural origin and after evaluating their compatibility with different actives, four specific compounds were selected for the continuation of the project:

- Fibrulose (FOS - oligosaccharide fruit) an oligosaccharide with the function of diluent/binder that is distinguished by its prebiotic function.
- Cristal Tex 626, tapioca maltodextrins with a lower glycemic index than classic maltodextrins, which have the function of a diluent/binding excipient.
- Compritol e ato, a lipophilic excipient, a substance that dissolves easily in oils and fats, which acts as a barrier to maximize the stability of the active ingredients in the tablet
- Fiberaid AG, larch arabinogalactan with the function of diluent and ancillary technological support with prebiotic action, immunomodulating, useful in metabolic control.

The second phase of the work involved the evaluation of the possibility of using these ingredients of natural origin in combination in specific ratios to totally and/or partially replace conventional excipients within mixtures containing different actives for the development of new nutraceutical products or for the restyling of products already marketed, in the form of coated, chewable and orosoluble tablets, in order to improve or enhance their technical and functional properties.

SUMMARY OF THE INVENTION

The Applicant has found that powders containing the aforementioned ingredients of natural origin indicated above have excellent characteristics and good properties in terms of tapped density, flowability and flow function in many cases better than those obtained using conventional excipients.

Object of the present invention are therefore tablets obtainable by compression of powder mixtures comprising:

a) At least one active ingredient,
b) At least one diluent;
c) At least one lubricating agent
d) At least one excipient selected from at least one of the following classes: acidifier, binder, disaggregating agent, anti-caking agent, sweetener, stabiliser

wherein:

i) the diluent b) is a mixture of at least one conventional diluent and fructo-oligosaccharides (FOS),
ii) the lubricating agent is a mono- and/or diglyceride of a fatty acid;
iii) the weight ratio FOS/conventional diluent is between 0.5 and 3, preferably between 0.7 and 2.5.

The tablets object of the present invention preferably also contain a component e) consisting of arabinogalactan when it is desired to impart immunomodulatory properties to the aforementioned tablets.

In this case (FOS+ arabinogalactan) /diluent between 0.7 and 3.5 preferably between 0.9 and 2.7.

Sono arrivata qui....

DESCRIPTION OF THE FIGURES

Figure 1 shows the equivalent diameters most commonly used in the pharmaceutical industry.

Figure 2 shows the flowability attitude based on the angle of repose.

Figure 2 shows the flowability attitude according to the Hausner index.

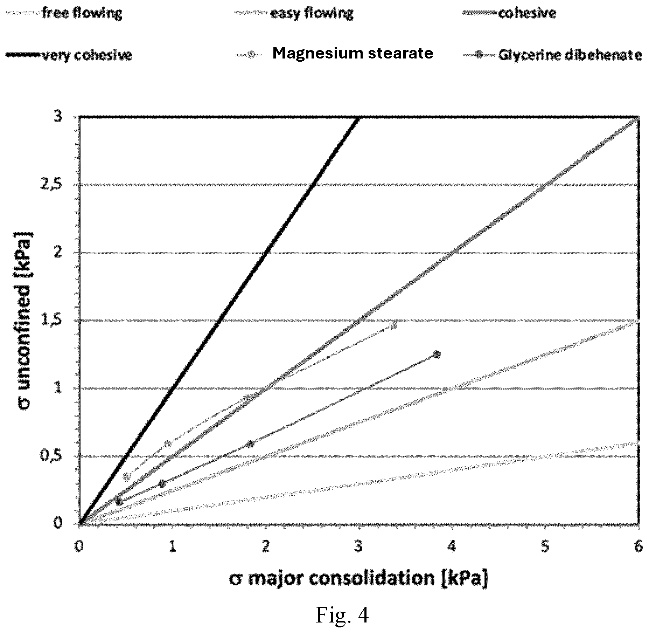

Figure 4 shows the flow function of magnesium stearate and mono- and/or diglycerylbeenate.

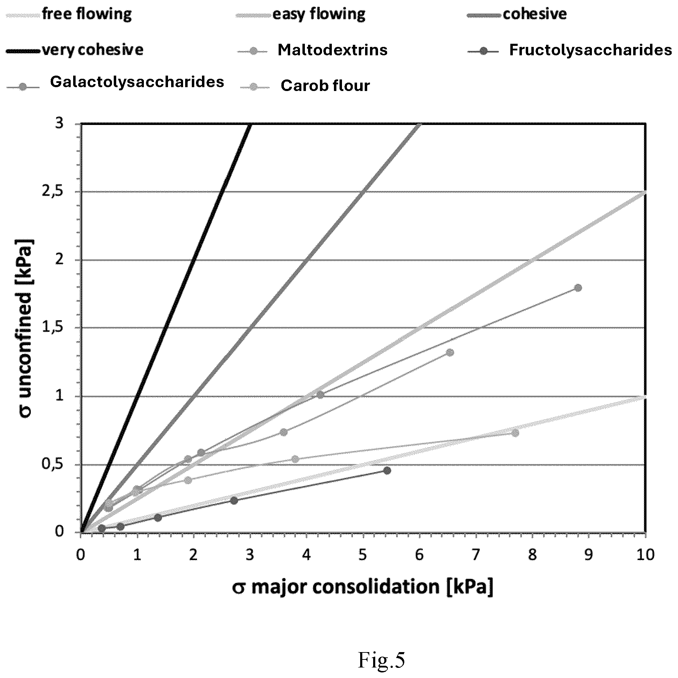

Figure 5 shows the flow function of the fructooligosaccharides.

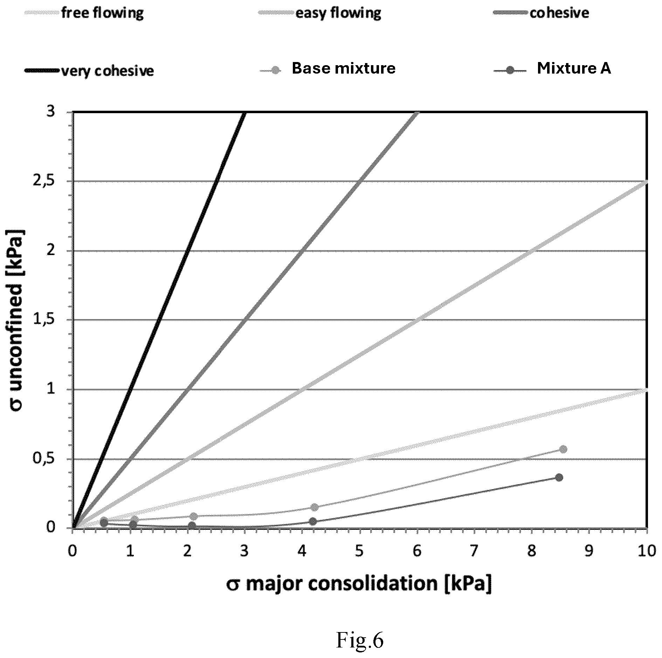

Figure 6 shows the flow function of the base mixture and mixture A shown in 2.1

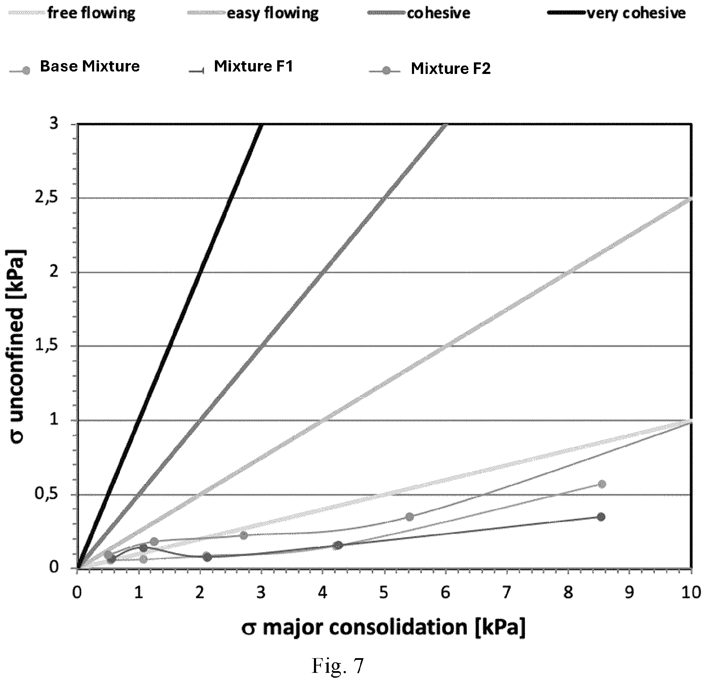

Figure 7 shows the flow function of the base mixture and mixtures F1 and F2 shown in 2.1.

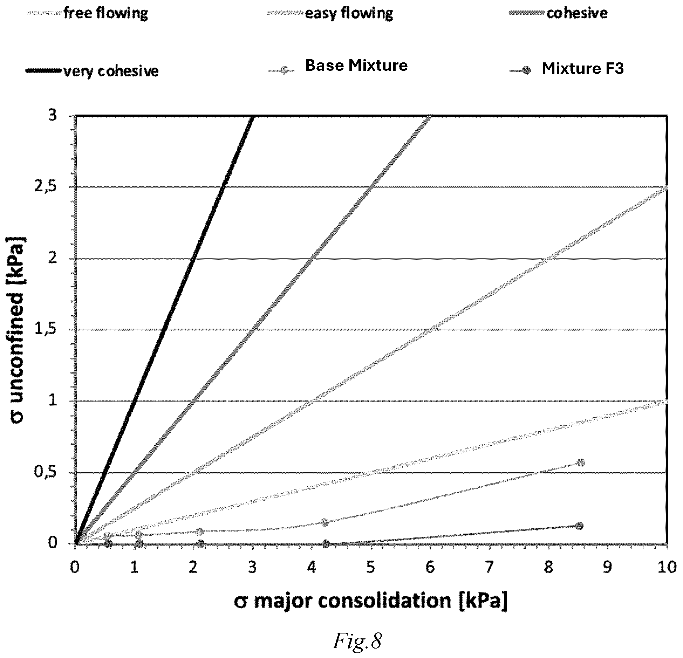

Figure 8 shows the flow function of the base mixture and mixture F3, shown in 2.1.

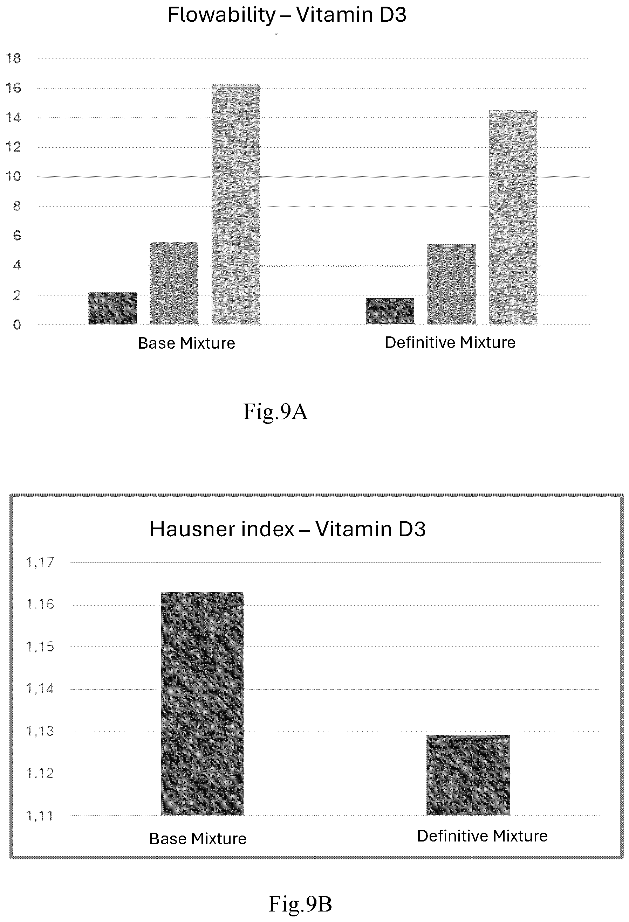

Figure 9 A shows the flowability of the complete mixture G2 vs. the flowability of the base mixture described in 2.1.

Figure 9 B shows the Hausner index of the complete Formula G2 vs. that of the base mixture described in 2.1.

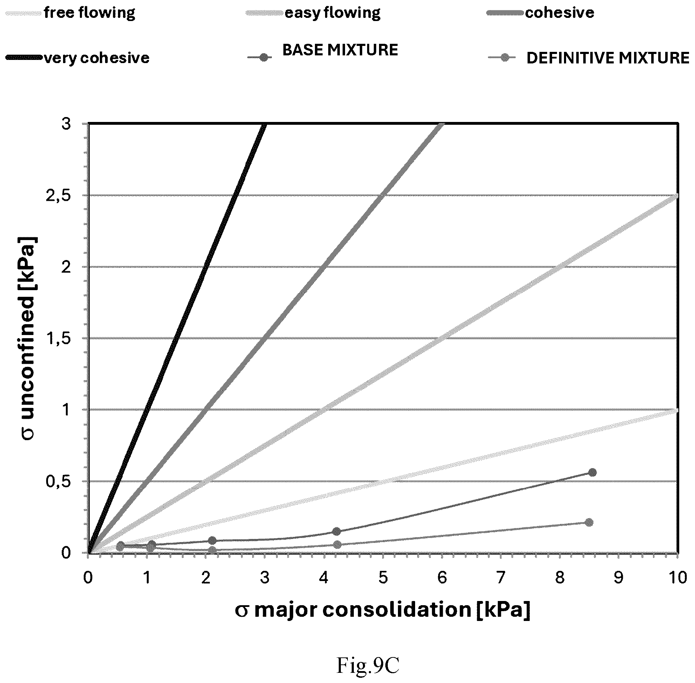

Figure 9 C shows the flow function of the complete mixture G2 vs. that of the base mixture described in 2.1.

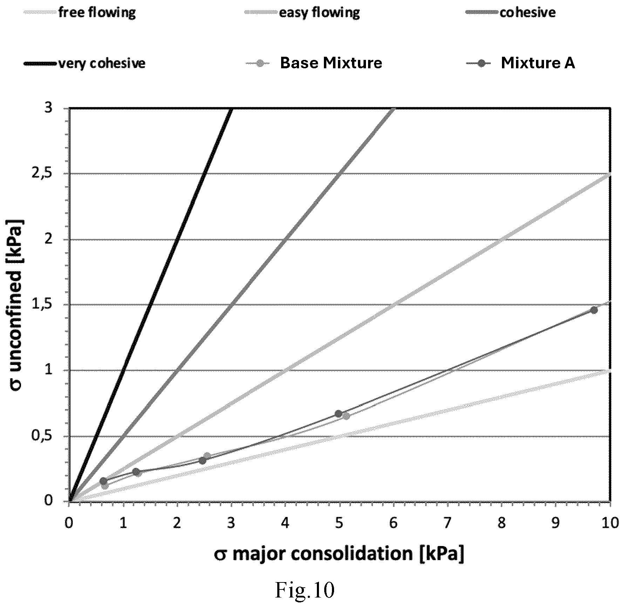

Figure 10 shows the flow function of the base mixture and that of mixture A described in 2.2.

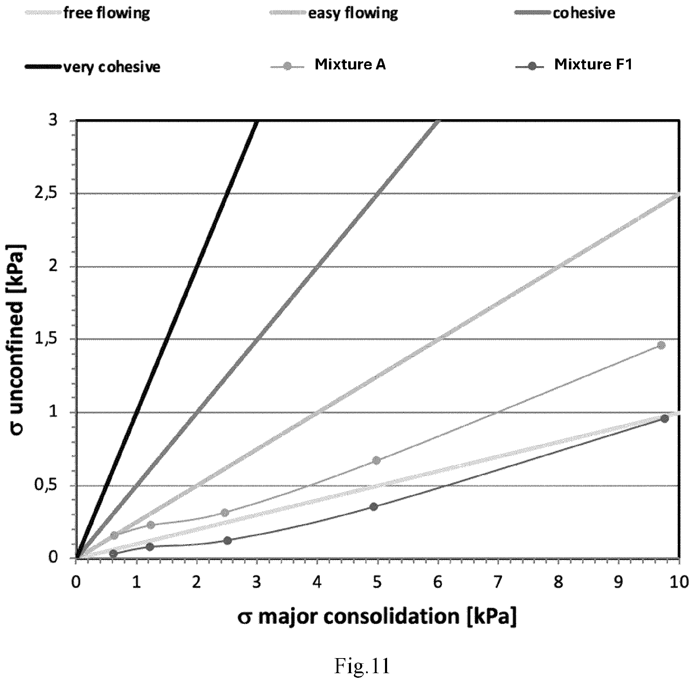

Figure 11 shows the flow function of the base mixture and of the mixture F1 reported in 2.2.

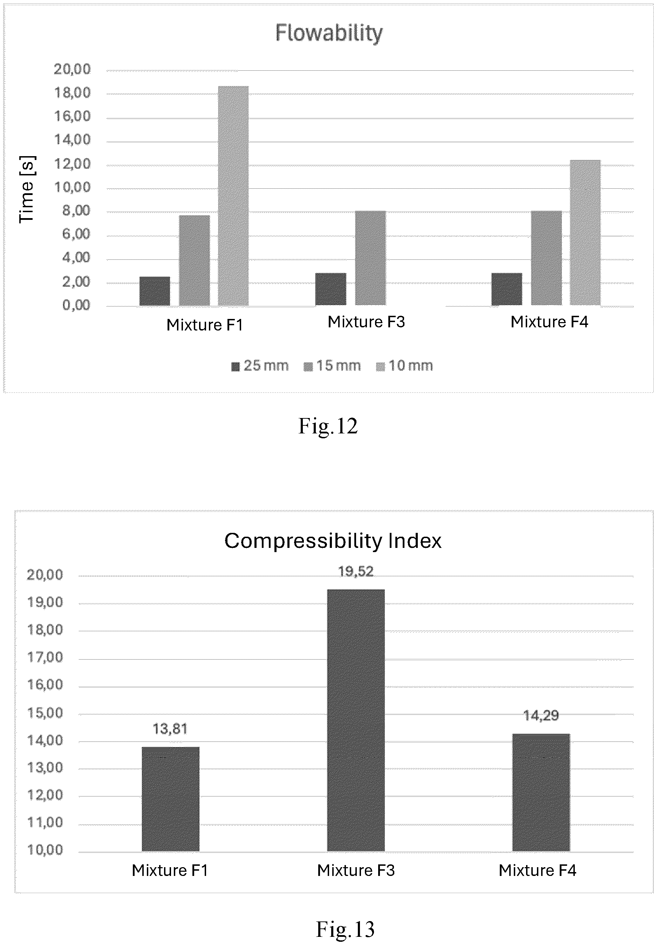

Figure 12 shows the flowability of mixtures F1, F3 and F4 described in 2.2.

Figure 13 shows the compressibility index of the mixtures F1, F3 and F4 described in 2.2.

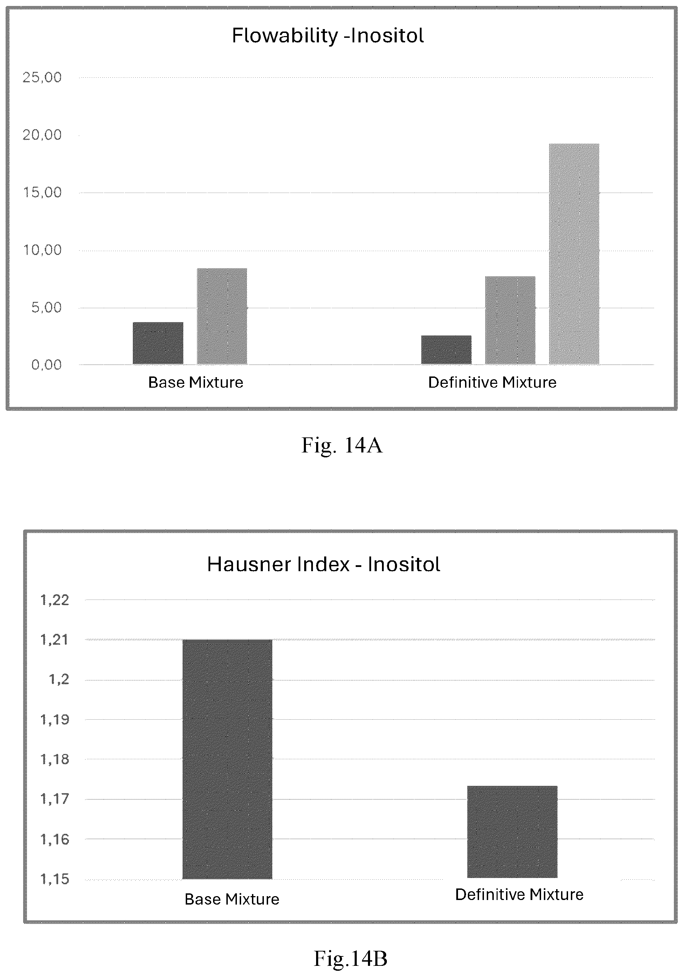

Figure 14 A shows the flowability of the final mixture C compared to the base mixture described in 2.2.

Figure 14 B shows the compressibility index of the final mixture C compared to the base mixture described in 2.2.

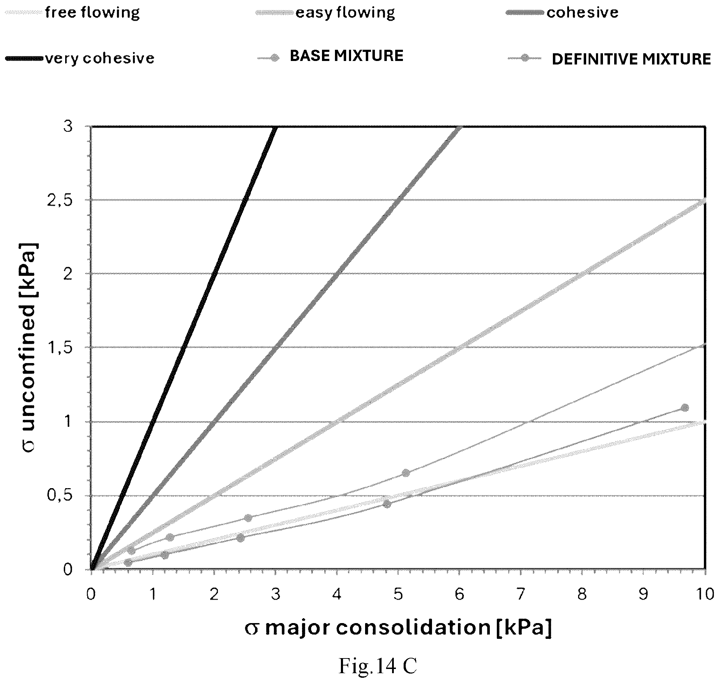

Figure 14 C shows the flow function of the final mixture C compared to the base mixture described in 2.2.

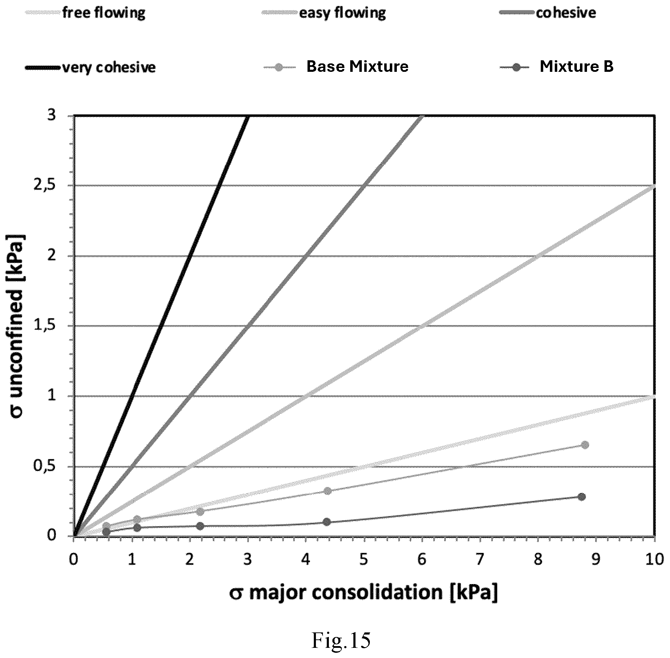

Figure 15 shows the flow function of the base mixture and mixtures A and B described in 2.3.

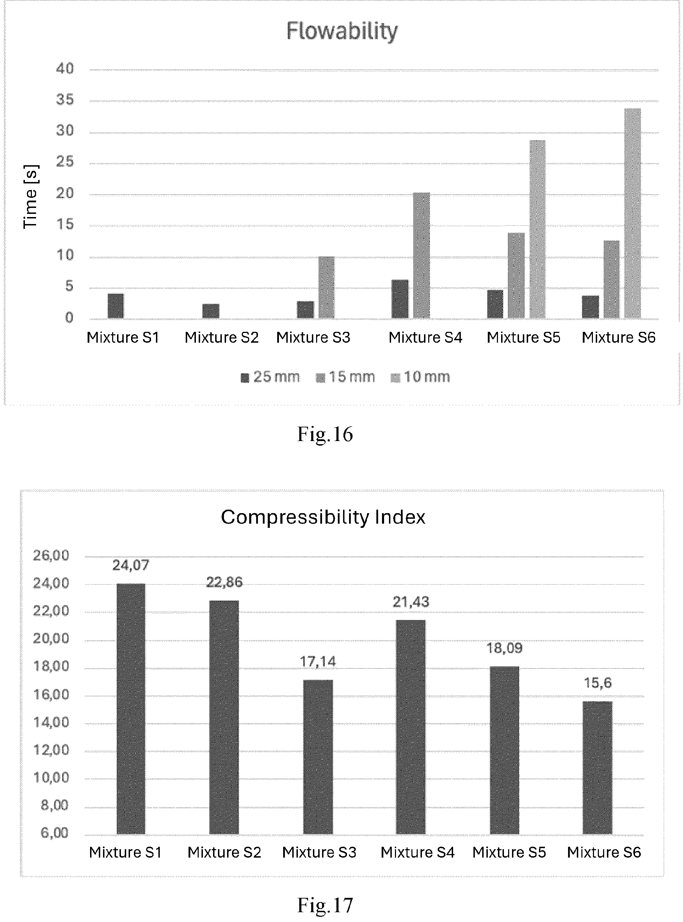

Figure 16 shows the flowability of mixtures S1, S2, S3, S3, S4, S5 and S6 described in 2.3.

Figure 17 shows the compressibility index of mixtures S1, S2, S3, S3, S4, S5 and S6 described in 2.3.

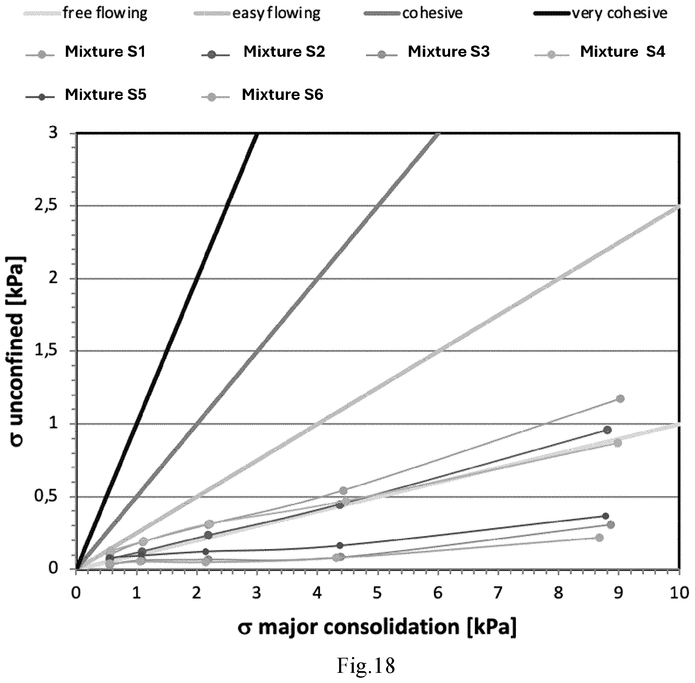

Figure 18 shows the flow functions of mixtures S1, S2, S3, S3, S4, S5 and S6 described in 2.3.

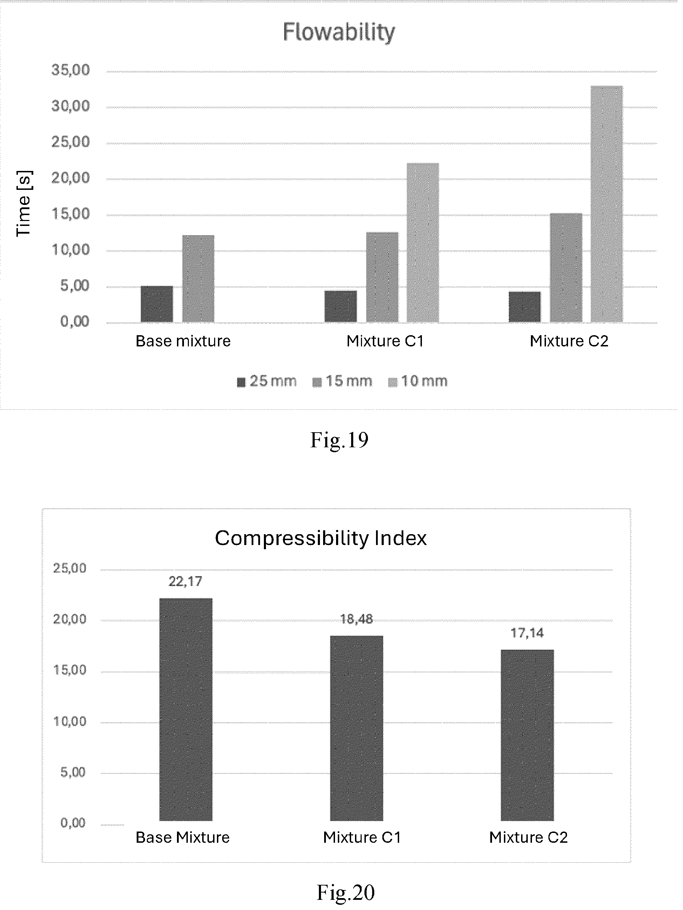

Figure 19 shows the flowability of the base mixture, of mixtures C1 and C2 described in 2.3.

Figure 20 shows the compressibility index of the base mixture and mixtures C1 and C2 described in 2.3.

Figure 21 A shows the flowability of formulations C1 and C2 described in 2.3.

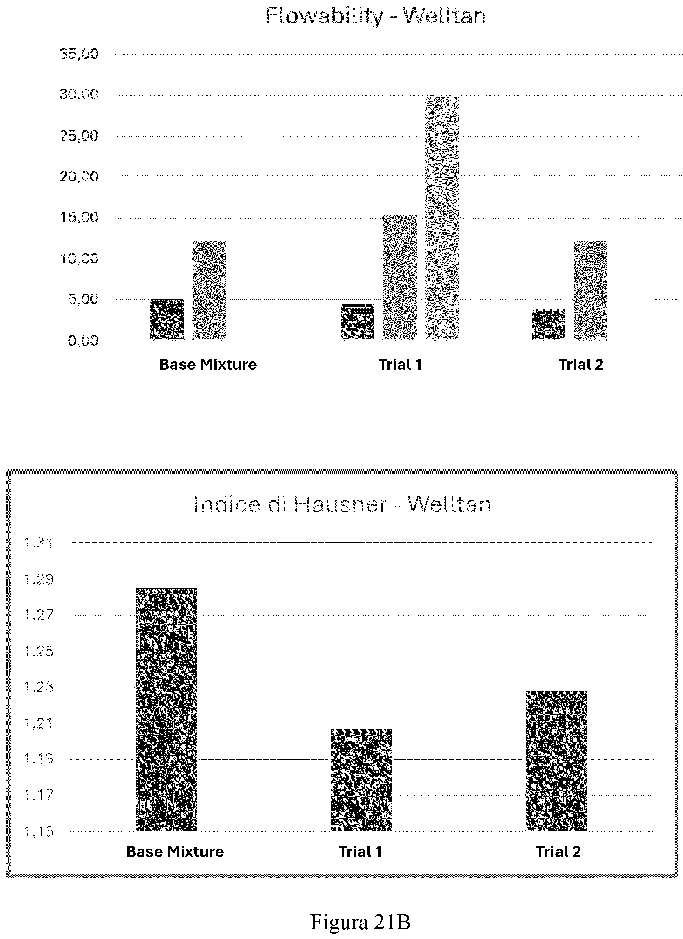

Figure 21 B shows the Hausner index of formulations C1 and C2 described in 2.3.

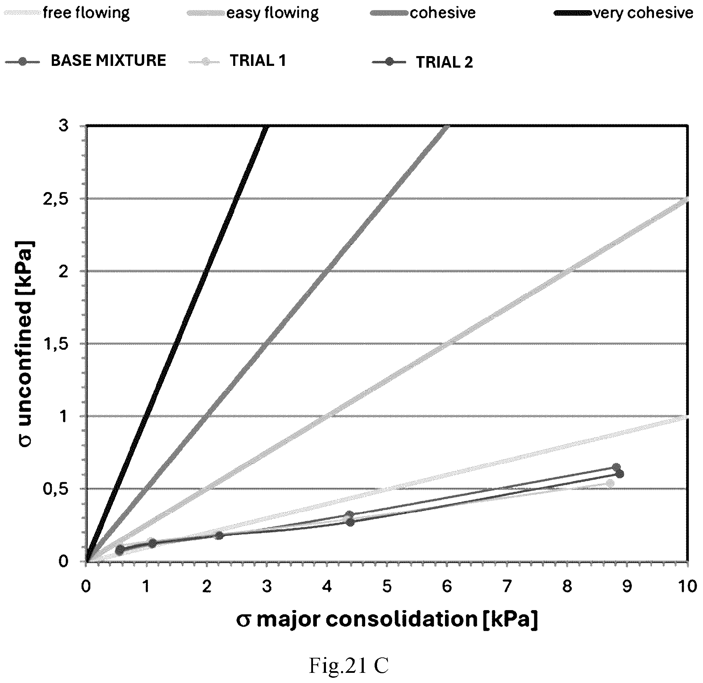

Figure 21C shows the flow function of the mixtures C1 and C2 described in 2.3.

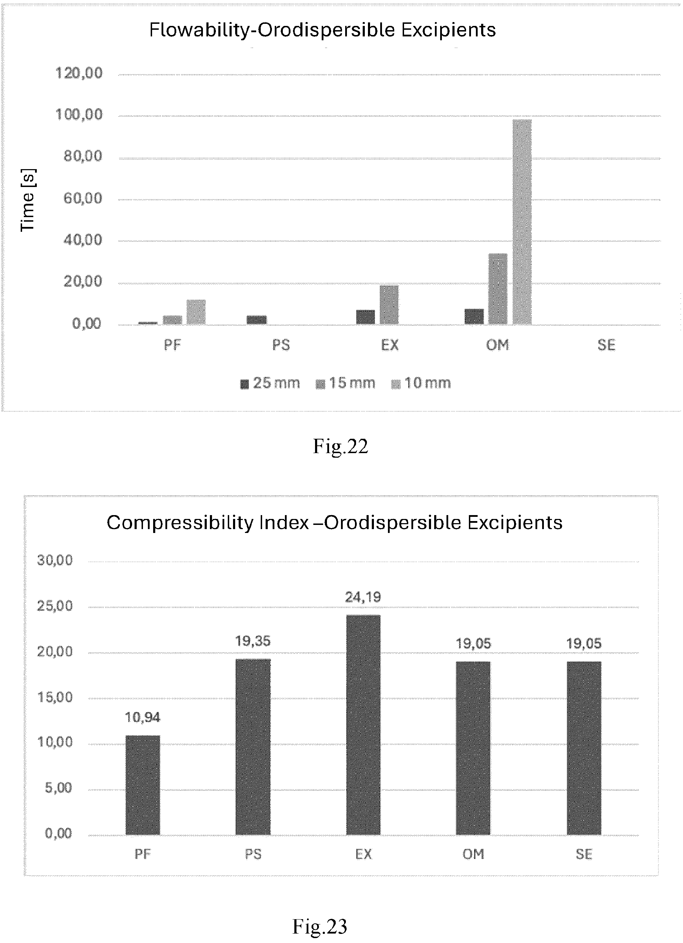

Figure 22 shows the flowability of some orodispersible excipients described in 2.4

Figure 23 shows the compressibility index of the orodispersible excipients described in 2.4.

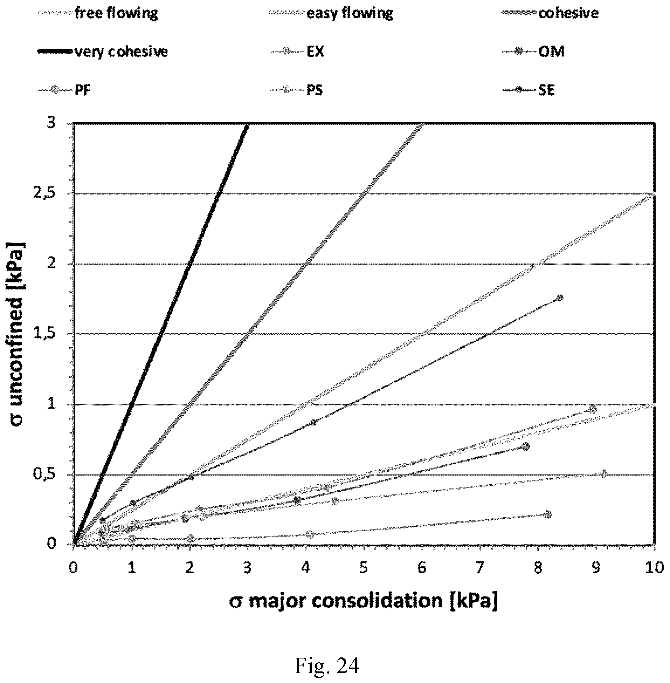

Figure 24 shows the flow function of the orodispersible excipients described in 2.4.

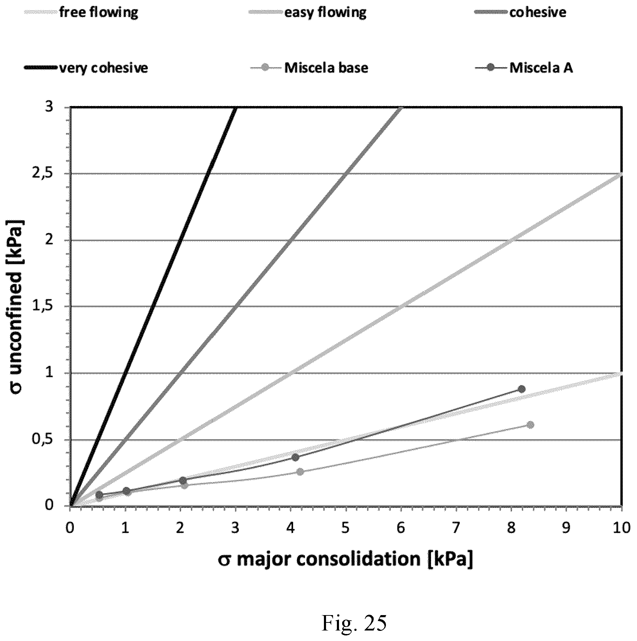

Figure 25 shows the flow function of mixture A compared to the base mixture described in 2.4.

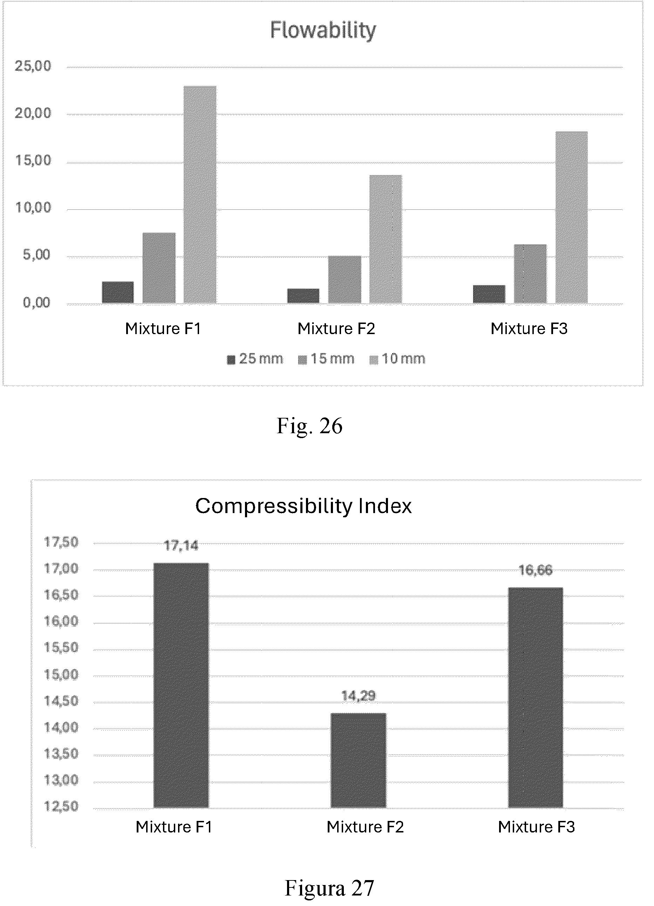

Figure 26 shows the flowability of the mixtures F1, F2 and F3 described in 2.4.

Figure 27 shows the compressibility index of the mixtures F1, F2 and F3 referred to in 2.4.

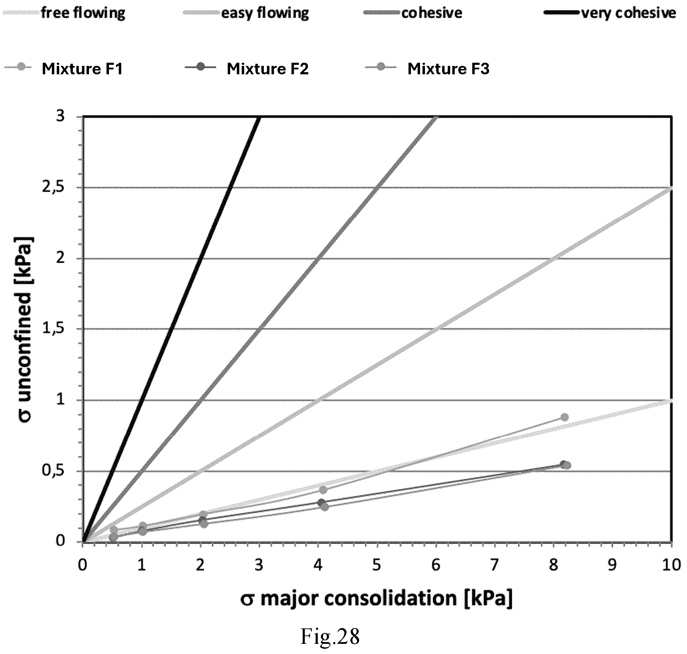

Figure 28 shows the flow function of the mixtures F1, F2 and F3 referred to in 2.4.

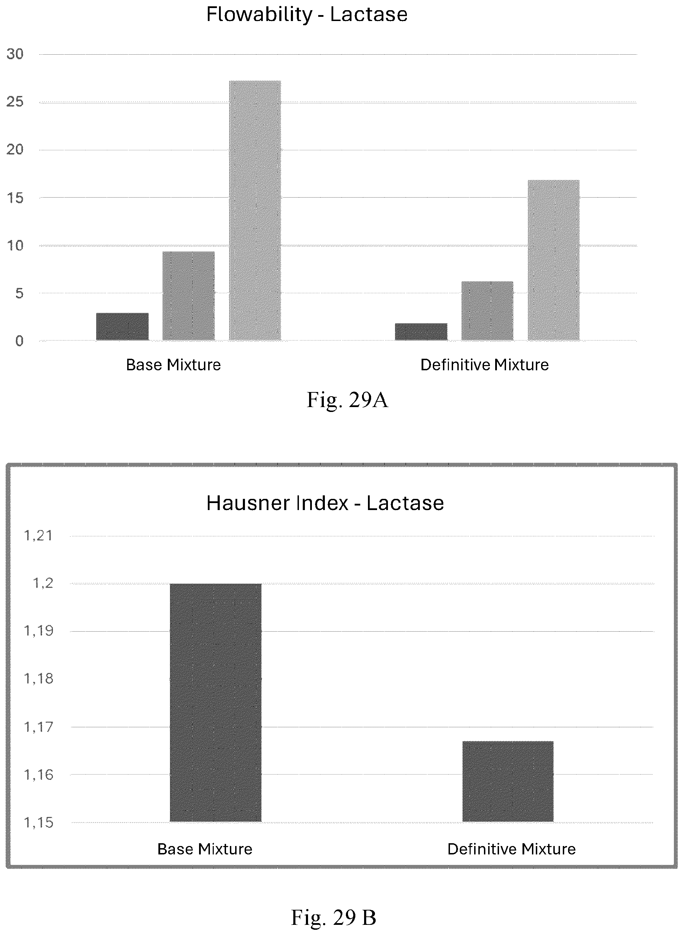

Figure 29 A reports the flowability of mixture C (definitive) vs. base mixture described in 2.4.

Figure 29 B shows the Hausner index of formulation C in comparison with the base mixture described in 2.4.

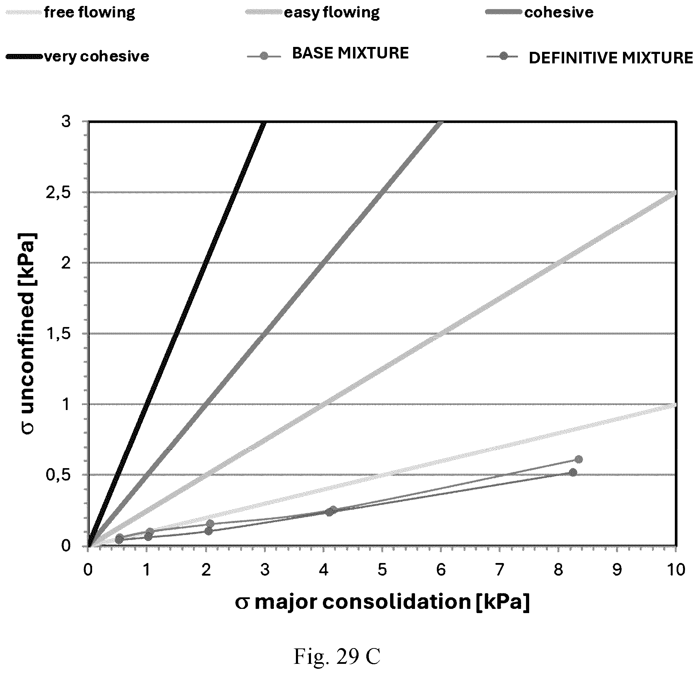

Figure 29 C shows the flow function of formula C in comparison with the base mixture contemplated in 2.4.

DETAILED DESCRIPTION OF THE INVENTION

For the purposes of the present invention, the definition "comprising" does not exclude the presence of other components than those expressly mentioned after said definition. For the purposes of this invention, the definitions "composed of" and "consisting of" exclude the presence of these additional components not expressly mentioned.

For the purposes of the present invention, the terms "ingredient of natural origin" or "alternative excipients " or "ingredients of natural origin with the function of a functional excipient" mean those ingredients of natural origin that the Applicant has analysed and selected to partially or completely replace some conventional excipients commonly used in the nutraceutical field for the formulation of solid forms (tablets and capsules) and that are distinguished by certain characteristics, such as technology, health properties, functionality and innovation.

The lubricant is preferably mono- and/or diglycerylbehenate or Glyceryl dibehenate.

The diluents of conventional type present in the powders contained in the tablets object of the present invention are preferably selected from at least one of: isomalt, mannitol, microcrystalline cellulose, calcium phosphate, sorbitol, xylitol, maltitol, isomaltulose, erythritol.

The binder when present in the tablets of the invention is hydroxypropylcellulose.

The anti-caking agent when present in the tablets of the invention is preferably selected from silicon dioxide and calcium carbonate.

The acidifier, when present, is preferably selected from tartaric acid, citric acid and ascorbic acid.

The disaggregating agent when present is preferably selected among a mixture of corn starch and mannitol, cross-linked sodium carboxymethylcellulose (CMC), polyvinylpolypyrrolidone (PVPP), starches and derivatives thereof.

The following particularly preferred embodiments of the tablets object of the present invention are reported.

A) chewable tablet, wherein the conventional diluent consists of a mixture of mannitol and isomalt and the ratio (FOS+ arabinogalactan)/ diluent is 1.14 while the mono- and/or diglycerylbeenate is present at concentrations comprised of 2.5 to 3% by weight on total weight of the tablets.

B) swallowable tablet containing as conventional diluent microcrystalline cellulose and possibly hydroxypropylcellulose as binder, wherein the weight ratio (FOS+ arabinogalactan)/conventional diluent is comprised between 1.11 and 2.45 while the content of mono- and/or di-glycerylbeenate is comprised between 2.75 and 3% by weight of total weight of the tablet.

C) Swallowable tablet containing microcrystalline cellulose as a conventional diluent, the anticaking agent consists of a mixture of silicon oxide in amorphous form with d50 measured with laser diffraction according to ISO 13320 of 320 $\mu$m and of silicon in amorphous form, with d50 measured with Malvern® Mastersizer diffractor comprised between 2.0 and 4 $\mu$m in 1:1 weight ratio, the weight ratio (FOS + arabinogalactan)/conventional diluent is comprised between 0.9 and 2.45, the mono- and/or diglycerylbeenate is present at concentrations of 3% by weight on the total weight of the tablet.

D) Orodispersible tablet, containing as conventional diluent crystalline cellulose and as disaggregating agent mannitol free of hydroxypropylcellulose as binder in which the weight ratio (FOS+arabinogalactan)/conventional diluent is 1.8 the mono- and/or diglycerylbeenate is present at concentrations of 1% by weight on the total weight of the tablet.

The entire experimental study that allowed the identification of the tablets object of the present invention is reported for illustrative purposes.

EXPERIMENTAL STUDY.

In this study rheology, as physical analysis, was used by the Applicant to characterize the individual ingredients of natural origin and the excipients in powder and mixtures, comparing their flowability properties after the application of external forces. In particular, thanks to the application of rheology, it was possible to select mixtures composed of ingredients of natural origin that differ from traditional excipients in health properties, functionality and innovation. The purpose is to study flowability properties thereof by comparing them with those of conventional excipients, in order to ensure compatibility with the system and avoid interference during the processing and production phases.

The study was therefore carried out for the purpose of characterizing the physical-mechanical properties of ingredients of natural origin that the Applicant intends to use in place of conventional excipients commonly used in the nutraceutical field for the formulation of solid forms (tablets and capsules), which are distinguished by certain characteristics, such as technology, health properties, functionality and innovation.

The work was divided into several steps: in the first step the ingredients of natural origin were compared with each other in terms of density and flowability; subsequently, synergistic associations were identified that were inserted in the formula in place of conventional excipients in order to optimize their technical and functional properties, avoiding inconveniences during the delicate phases of production, quality control and packaging.

For each ingredient and each mixture, the flowability was initially evaluated through the use of the *flowability tester* and the evaluation of the Hausner and compressibility indices through the use of the *tapped density tester.*

Secondly, the samples were analysed with the use of the *shear cell.* Rheological analysis was useful to understand the behaviour of each individual powder and mixtures, verify their flowability and compare the results with those obtained with the other instruments used.

The first part of the work was focused on the characterization of each excipient, both conventional and those of natural and functional origin. Excipients and ingredients of natural origin having similar functions and technological properties were then compared. Each individual excipient and ingredient of natural origin was analysed using the following instruments: *flowability tester, tapped density tester, Shear cell rheology and Differential Scanning Calorimetry (DSC).*

PART 1 CHARACTERIZATION OF TRADITIONAL EXCIPIENTS AND INGREDIENTS OF NATURAL ORIGIN WITH THE FUNCTION OF FUNCTIONAL EXCIPIENT

Magnesium stearate is the magnesium salt of fatty acids obtained from oils and fats used in the food industry. It is a white powder, very fine, insoluble in water and greasy to the touch, used as an anti-caking agent and lubricant.

The plant-based excipient Compritol e ato (glyceryl dibehenate), a mixture of mono-, di- and tri-glycerides of behenic acid, were used instead of magnesium stearate. It is a lipophilic excipient that, in addition to improving the flowability and wettability of poorly soluble actives, acts as a barrier to maximize the stability of the actives in the formulation. In addition, in the formulation of a tablet, at concentrations greater than 10% it may be useful for modulating the release of the active ingredient.

In Table 1, it can be seen that both excipients do not flow to any diameter of the flowability tester; instead, it can be observed that the excipient glyceryl dibehenate has a lower Hausner and compressibility indexes than magnesium stearate. From this it is evident that magnesium stearate has a lower flowability compared to *glyceryl dibehenate,* ranging from a "poor" flowability in the case of magnesium stearate to a "discrete/acceptable" flowability for glyceryl dibehenate.

*Table 1: results obtained from the flowability tester and the tapped density tester*

| Excipient | Flowability | | | H.I. | C.I. |
|---|---|---|---|---|---|
| | 25 mm | 15 mm | 10 mm | | |
| Magnesium stearate | no | no | no | 1,38 | 27,42 |
| Glyceril dibehenate | no | no | no | 1,25 | 19,67 |

Figure 4 shows the flow functions of the two excipients: it can be noted that magnesium stearate has a low *consolidation stress* trend located in the very cohesive zone to conclude in cohesive at high consolidation stresses, while glyceryl dibehenate is located in the cohesive range, thus highlighting a better flowability compared to magnesium stearate.

Among the powders used within the mixtures as diluent excipients, bulking agents and/or stabilizers we find inorganic compounds such as silicon dioxide and calcium phosphate which also acts as an acidity regulator, sweeteners such as isomalt and mannitol, and cellulose derivatives among which the most widespread are microcrystalline cellulose, hydroxypropylcellulose and carboxymethylcellulose which also perform the function of a disaggregating agent.

Several alternative excipients were used in place of these compounds, such as:

- FOS fructo-oligosaccharides that, in addition to acting as a diluent/binder, also perform a prebiotic function
- GOS galacto-oligosaccharides also have a prebiotic function

- carob flour, an alternative gum that performs a mucoadhesion function within the intestinal walls, modulating the release of the active ingredient
- tapioca maltodextrins, also used as diluents, which have a lower glycemic index than the maltodextrins normally used
- the larch arabinogalactans, used as ancillary diluents and with prebiotic action, which have an immunomodulatory action and are useful in metabolic control.

In Table 2, it can be observed that the FOS have more optimal values than the other compounds, with an "excellent" flowability attitude, as evidenced by the values assumed by the Hausner and compressibility indexes. For this reason, this ingredient was selected as a diluent/binder to replace conventional excipients.

*Table .2: results obtained at the flowability tester and the tapped density tester.*

| Excipient | Flowability | | | H.I. | C.I. |
|---|---|---|---|---|---|
| | 25mm | 15mm | 10mm | | |
| Fruit-oligosaccharides | yes | yes | yes | 1.11 | 10 |

| Excipient | | | | | |
|---|---|---|---|---|---|
| Galacto-oligosaccharides | no | no | no | 1.23 | 18.78 |
| Carob flour | no | no | no | 1.30 | 22.95 |
| Maltodextrins from tapioca | no | no | no | 1.22 | 18.33 |

Also in figure 5, the flow function of the fruit-oligosaccharides is the best with high flowability properties, in the range of free-flowing powders.

Subsequently, the excipients commonly used with diluent/binding function were also characterized and compared with the innovative functional excipient FOS.

The flowability tester (table 3), highlights the good flowability of only three excipients, namely calcium phosphate, FOS and Sipernat silicon dioxide. The microcrystalline cellulose, on the other hand, only flows at a diameter of 25 mm. All other excipients, both innovative and conventional, do not flow to any opening.

The data obtained with the use of the tapped density tester made it possible to significantly highlight the differences between the types of excipients. According to the guidelines of the Italian Official Pharmacopoeia and XII, microcrystalline cellulose, carboxymethylcellulose have a "poor" flowability attitude; hydroxypropylcellulose and Syloid silicon dioxide "discrete/acceptable", calcium phosphate and Sipernat silicon dioxide "excellent".

*Table 3: results obtained from the flowability tester and the tapped density tester.*

| Excipient | Flowability | | | H.I. | C.I. |
|---|---|---|---|---|---|
| | 25mm | 15mm | 10mm | | |
| Microcrystalline cellulose | yes | no | no | 1.36 | 26.47 |
| Carboxymethylcellulose | no | no | no | 1.40 | 28.33 |
| Calcium phosphate | yes | yes | yes | 1.11 | 10 |
| Hydroxypropylcellulose | no | no | no | 1.22 | 18.09 |
| Silicon dioxide Syloid | no | no | no | 1.20 | 16.39 |
| Silicon dioxide Sipernat | yes | yes | yes | 1.09 | 8.57 |
| Fruit-oligosaccharides | yes | yes | yes | 1.11 | 10 |

The rheometer analysis shows that the only excipients with a flow function located within the free-flowing range are silicon dioxide sipernat, calcium phosphate and microcrystalline cellulose. Carboxymethylcellulose is found in the easy flowing powder zone. Syloid silicon dioxide is located in the area of cohesive powders, while hydroxypropylcellulose is located in the very cohesive area.

PART 2 - CHARACTERIZATION OF POWDERS TO BE COMPRESSED

*2.1 Mixture 1*

Mixture 1, consisting of 10% of active ingredient, is intended for the formulation of chewable tablets with the function of supporting the immune system and useful in maintaining normal muscle function. It consists of isomalt and mannitol with the function of diluents at a total concentration of 87%, tartaric acid used as an acidifier, 1.5% magnesium stearate and 1% silicon dioxide as lubricants and anti-caking agents.

*Table 4 - Formula of mixture 1*

| Excipient | % w/w |
|---|---|
| Active | 10 |
| Diluents | 87 |
| Acidulant | 0.5 |
| Anti-caking agents | 2.5 |

For these mixtures, systematic analyses were carried out in which the excipients commonly used were partially or totally replaced with the innovative ones.
The first step was based on the comparison between the mechanical properties of the base mixture and mixture A in which magnesium stearate was replaced with glyceryl dibehenate, an alternative excipient that, in addition to having a lubricating action, guarantees greater stability to the active ingredient (table .5).

*Table 5: replacement of magnesium stearate with glycerin dibehenate.*

| | % w/w | |
|---|---|---|
| **Excipient** | **Base mixture** | **Mixture A** |
| Magnesium stearate | 1.5 | - |
| **Glyceryl dibehenate** | **-** | **1.5** |

The flowability results obtained with the flowability tester (table 3.6), do not show particular differences between the base mixture and mixture A, as well as the Hausner index and the compressibility index remain very similar. Both mixtures have a flowability attitude defined by the guidelines of the Official Pharmacopoeia as "discreet".

*Table 6: results obtained from the flowability tester and the tapped density tester.*

| | *Flowability* | | | *H.I.* | *C.I.* |
|---|---|---|---|---|---|
| | *25mm* | *15mm* | *10mm* | | |
| *Base mixture* | *yes* | *yes* | *yes* | *1.16* | *14.00* |
| *Mixture A* | *yes* | *yes* | *yes* | *1.16* | *13.81* |

A further confirmation is given by the rheological analysis carried out on the two mixtures (Figure 6), in which it can be noted that mixture A has a slightly lower flow function than the curve of the base mixture, even if both are located in the "free flowing" range.

Subsequently, mixtures were formulated in which, in addition to the replacement of the lubricant, a complete replacement of the two main excipients with the function of diluents was carried out, with different percentages of the innovative excipients with prebiotic functionality, fructo-oligosaccharides and tapioca maltodextrins (table 7).

*Table 7: total replacement with fruit oligosaccharides and tapioca maltodextrins.*

| | % w/w | | |
|---|---|---|---|
| **Excipient** | **Base mixture** | **Mixture F1** | **Mixture F2** |
| **Isomalt** | **69** | **-** | **-** |
| **Mannitol** | **18** | **-** | **-** |
| **Fructo-oligosaccharides** | **-** | **87** | **77** |
| Maltodextrins from tapioca | - | - | 10 |
| Glyceryl dibehenate | - | 1.5 | 1.5 |

The mixture F1 flows at all three diameters to the flowability tester, while the mixture F2 does not flow at 10 mm.

In the tapped density tester, on the other hand, the Hausner index and the compressibility index are very similar in both mixtures, with a slight improvement in the mixture F1 (Table 8).

*Table 8: results obtained from the flowability tester and the tapped density tester.*

| | Flowability | | | H.I | C.I |
| --- | --- | --- | --- | --- | --- |
| | **25mm** | **15mm** | **10mm** | | |
| Base mixture | yes | yes | yes | 1.16 | 14.00 |
| Mixture F1 | yes | yes | yes | 1.11 | 10.14 |
| Mixture F2 | yes | yes | no | 1.12 | 11.59 |

The curves obtained by rheological analysis (Figure 7) show that the presence of tapioca maltodextrins decreases the flowability properties of the mixture, compared to the use of only the fructo-oligosaccharides in the mixture F1. Both are however located in the free flowing range.

In the subsequent mixtures, only the fructo-oligosaccharides were used as innovative excipients to be replaced for those commonly used, as it was possible to observe that the presence of tapioca maltodextrins does not bring any improvement to the flowability properties, indeed it would seem to worsen them.

The mixture F1, despite having a "good" flowability attitude, during the compression tests determined the descaling phenomenon in which compliant tablets could not be obtained, probably due to the excessive flowability and hygroscopicity of the fructo-oligosaccharides.

For this reason, a mixture has been formulated in which some of the main and most commonly used excipients have been partially replaced with the function of diluent, present in high concentrations within the starting mixture, namely isomalt and mannitol (Table 9).

*Table 9: partial replacement of common excipients with fructo-oligosaccharides.*

| | % w/w | |
| --- | --- | --- |
| **Excipient** | **Base mixture** | **Mixture F3** |
| Isomalt | 69 | 34.5 |
| Mannitol | 18 | 9 |
| **Fructo-oligosaccharides** | - | **43.5** |
| Glyceryl dibehenate | - | 1.5 |

Although both mixtures flow at all three openings to the flowability tester, the Hausner index and the compressibility index are better in the mixture F3 in which there is a partial replacement of the excipients with the FOS, going from a "discreet" to a "good" flowability attitude (table 10).

*Table 10: results obtained from the flowability tester and the tapped density tester.*

| | Flowability | | | H.I. | C.I. |
| --- | --- | --- | --- | --- | --- |
| | **25mm** | **15mm** | **10mm** | | |
| Base mixture | yes | yes | yes | 1.16 | 14.00 |
| Mixture F3 | yes | yes | yes | 1.13 | 11.43 |

Rheological analysis (Figure 8) shows that the mixture F3 has better flowability properties than the base mixture. For this reason, these concentrations of common excipients and innovative excipients have been confirmed in order to be able to introduce a further ancillary functional excipient, the arabinogalactans, which have an immunomodulatory function (Table 11).

*Table 11: addition of arabinogalactans*

| Excipient | % w/w | |
| --- | --- | --- |
| | Mixture F3 | Mixture F4 |
| Isomalt | 34.5 | 32 |
| Mannitol | 9 | 9 |
| Fructo-oligosaccharides | 43.5 | 43.5 |
| Glyceryl dibehenate | 1.5 | 1.5 |
| Arabinogalactans | - | 2.5 |

At both flowability tester and tapped density tester, both mixtures do not show significant differences, maintaining good flowability properties, both with "good" flowability.

At both flowability tester and tapped density tester, both mixtures do not show significant differences, maintaining good flowability properties, both with "good" flowability.

*Table 12: systematic analyses with different concentrations of glycerin dibehenate.*

| Excipient | % w/w | | |
| --- | --- | --- | --- |
| | Mixture F4 | Mixture G1 | Mixture G2 |
| Diluents | 41 | 40.5 | 40.5 |
| Fructo-oligosaccharides | 43.5 | 43.5 | 43.5 |
| Glycerin dibehenate | 1.5 | 2 | 2.5 |
| Arabinogalactans | 2.5 | 2.5 | 2.5 |

In particular, in the mixture G2 whose complete formulation also of active ingredient is shown in the following table 13, 48.5% of the formula (53.8% without active ingredient) of the base mixture has been modified. The formulation with active ingredient is shown in table 14

In the reformulated mixture 54% isomalt and 50% mannitol was replaced by FOS + Fiberaid. (in the final formula fos+fiberaid :isomalt+mannitol 1.14:1

*Table 13 complete formulation corresponding to formulation G2*

| Complete mixture G2 | % |
|---|---|
| Vitamin D3 0.25% | 10 |
| Isomalt | 31.25 |
| Mannitol | 9 |
| **FOS** | **43.5** |
| **Compritol e ato** | **2.5** |
| **Fiberaid** | **2.5** |
| Tartaric acid | 0.25 |
| Silica | 1 |

*Table 14 Base mixture A complete*

| Complete BASE MIXTURE | % |
|---|---|
| Vitamin D3 0.25% | 10 |
| Isomalt | 69 |
| Mannitol | 18 |
| Tartaric acid | 0.5 |
| Mg stearate | 1.5 |
| Silica | 1 |

The flowability is shown in Figures 9A, 9B and 9C respectively. The Hausner index and the flowing function of the formulation G2 (final mixture) compared to those of the base mixture.

As can be seen from the graphs, the mixture G2 flows better than the base mixture.

Formulation G3 for chewable tablet in which the active ingredient is vitamin B12 was also evaluated as reported in the following table 15 in comparison with the base mixture reported in table 16.

*Table 15 formulation for vitamin B12 chewable tablet*

| FORMULATION G3 | % |
|---|---|
| Vitamin B12 98% | 0.56 |
| Isomalt | 39.44 |
| Mannitol | 9 |
| FOS | 43.5 |
| Compritol e ato | 3 |
| Fiberaid | 2.5 |
| Silica | 1 |
| Flavouring | 1 |

*Table 16*

| BASE MIXTURE | % |
|---|---|
| Vitamin B12 98% | 0.56 |
| Isomalt | 77.94 |
| Mannitol | 18 |
| Mg stearate | 1.5 |
| Silica | 1 |
| Flavouring | 1 |

In the mixture G3 49% of isomalt and 50% of mannitol were replaced by FOS + galactomannans (fiberaid) in the final formula the weight ratio (FOS+ galactomannans)/(isomalt + mannitol) is 0.95:1.

*2.2. Mixture 2*

Mixture 2, consisting of 42.52% of active ingredient, is intended for the formulation of swallowable tablets with the function of rebalancing the body's tissues, activating cell cleaning processes (Table 17). It consists of microcrystalline cellulose and calcium phosphate at a total concentration of 49.23% having the function of diluents, 5% hydroxypropylcellulose acting as a binder, 2% glyceryl dibehenate and 0.75% magnesium stearate with lubricating function and, finally, 0.5% silicon dioxide used as an anti-caking agent.

*Table 17 base mixture*

| Excipient | % w/w |
|---|---|
| Active | 42.52 |
| Diluent | 49.23 |
| Binder | 5 |
| Lubricant | 2 |
| Anti-caking agent | 1.25 |

Systematic analyses were carried out in which a partial or total replacement of the conventional excipients was carried out. As a first step, magnesium stearate was replaced by glyceryl dibehenate, formulating a mixture containing 2.75% (Table 18).

*Table 18: replacement of magnesium stearate with glyceryl dibehenate.*

| | % w/w | |
|---|---|---|
| Excipient | Base mixture | Mixture A |
| Magnesium stearate | 0.75 | - |
| **Glyceryl dibehenate** | 2 | 2.75 |

In Table 19, it can be seen that mixture A has a lower flowability than the base mixture, probably due to a very high percentage of *glyceryl dibehenate*. In addition, in mixture A, the Hausner index and the compressibility index are high, going from a flowability discreet to an acceptable attitude.

*Table 19: results obtained from the flowability tester and the tapped density tester.*

| | Flowability | | | H.I. | C.I. |
|---|---|---|---|---|---|
| | 25mm | 15mm | 10mm | | |
| Base mixture | yes | yes | no | 1.21 | 17.31 |
| Mixture A | yes | no | no | 1.26 | 20.77 |

In Figure 10, the flow functions are quite overlapping and both are located in the *"easy flowing range"*. From these results we can deduce that the total replacement of magnesium stearate with glyceryl dibehenate does not implement the flow properties of the mixture, indeed it worsens them and it is necessary to combine them with other agents facilitating flowability of the powder.

Subsequently, possible synergistic actions between the new excipients and *glyceryl dibehenate* were evaluated, formulating mixtures that contained a partial replacement of some common excipients with the innovative ones: specifically, calcium phosphate, one of the two diluents present in the formula, and hydroxypropylcellulose with a binder function, were replaced with FOS (Table 20).

*Table 20: partial replacement of common excipients with innovative excipients.*

| | % w/w | |
|---|---|---|
| **Excipient** | **Mixture A** | **Mixture F1** |
| Microcrystalline cellulose | 29.23 | 29.62 |
| Calcium phosphate | 20 | - |
| Hydroxypropyl cellulose | 5 | - |
| **Glycerin dibehenate** | **2.75** | **2.75** |
| **Fructo-oligosaccharides** | **-** | **24.61** |

In Table 21, it is observed that mixture A only flows at the diameter of 25 mm, while mixture F1 flows at all openings of the flowability tester.

The compressibility index is significantly lower in the mixture F1, leading to a "good" flowability compared to the "acceptable" flowability of the base mixture.

*Table 21: results obtained from the flowability tester and the tapped density tester.*

| | Flowability | | | H.I | C.I |
|---|---|---|---|---|---|
| | 25mm | 15mm | 10mm | | |
| Mixture A | yes | no | no | 1.26 | 20.77 |
| Mixture F1 | yes | yes | yes | 1.16 | 13.81 |

Also, in the graph obtained from the rheological analysis, it is evident that the partial replacement of the common excipients with the fructo-oligosaccharides significantly improves the flowability properties of the mixtures. In particular, the base mixture has a *flow function* located within the easy flowing range, while the mixture F1 has a curve within the *"easy flowing"* area (Figure 11).

It is evident that the partial replacement of some diluent excipients and the total replacement of the binding excipients with fructo-oligosaccharides makes the mixtures certainly more fluid; however, the mixture F1, during the compression tests, exhibited the phenomenon of descaling and also the tablets obtained were inhomogeneous in the friability and abrasion tests.

For this reason, some diluent and binding excipients were reintroduced in order to find the optimal percentage to be used to obtain homogeneous tablets in all tests (Table 22).

*Table 22: systematic analyses with different percentages of microcrystalline cellulose and calcium phosphate.*

|  | % w/w | | | |
|---|---|---|---|---|
| **Excipient** | **Mixture F1** | **Mixture F2** | **Mixture F3** | **Mixture F4** |
| Microcrystalline cellulose | 29.62 | 24.62 | 14.62 | 19.62 |
| Calcium phosphate | - | - | 10 | - |
| Hydroxypropylcellulose | - | 5 | 5 | 5 |
| **Glyceryl dibehenate** | **2.75** | **2.75** | **2.75** | **2.75** |
| **Fructo-oligosaccharides** | **24.61** | **24.61** | **24.61** | **29.61** |

From the results obtained with *the flowability tester* and the *tapped density tester,* it was observed that the mixture F2, despite the re-insertion of the binder, did not flow at the flowability tester and, for this reason, in the next mixture F3, the 10% calcium phosphate was also reintroduced, however, obtaining a mixture that does not flow to the smallest opening of the funnel (figure 12).

In the mixture F4, finally, the percentage of microcrystalline cellulose and fructo-oligosaccharides was increased, while calcium phosphate was eliminated as it did not lead to any improvement in flowability. In fact, the compressibility index in the mixture F3 is very high compared to the other mixtures, with a "discrete" flowability attitude, compared to the "good" one of the mixtures F1 and F3 (figure 13).

On the basis of the results obtained from the previous systematic analyses, a final mixture was identified that had better flow properties in the *flowability tester, tapped density tester,* rheometer, in the tablet press and in the friability and abrasion tests, compared to the starting mixture. Finally, another excipient with an ancillary diluent function was added, namely the arabinogalactans from Larch , defined as formula C and reported in the following table 23

*Table 23 complete formulation C.*

| FORMULATION C | |
|---|---|
| **Component** | **%** |
| Myo Inositol | 42.52 |
| Microcrystalline cellulose | 19.62 |
| **FOS** | **27.11** |
| Hydroxypropylcellulose | 5 |
| **Compritol e ato** | **2.75** |
| **Fiberaid** | **2.5** |
| Silica | 0.5 |

When compared to the respective complete base formula shown in Table 24

*Table 24 complete base formula*

| BASE MIXTURE | |
|---|---|
| **Component** | **%** |
| Myo Inositol | 42.52 |
| Microcrystalline cellulose | 29.23 |
| Calcium phosphate | 20 |
| Hydroxypropylcellulose | 5 |
| Compritol e ato | 2 |
| Mg stearate | 0.75 |
| Silica | 0.5 |

We observe that 30% of formulation C has been modified (56.4% without active ingredient).

All calcium phosphate was replaced, while 32.9% of the microcrystalline cellulose was replaced with FOS and arabinogalactans.

The ratio (FOS+ arabinogalactans)/microcrystalline cellulose is 1.51:1 the ratio of Mg stearate/glyceryldibeenate is 1:1.

Figures 14A, 14B and 14C respectively show the flowability the Hausner index and flow function of mixture C (final mixture) compared to the Base mixture.

The final mix flows better than the base mixture

*Mixture 3*

Mixture 3, consisting of 24% of active ingredient, is intended for the formulation of tablets with the function of maintaining a healthy balance of the microbiota and enhancing its functionality. It consists of microcrystalline cellulose and calcium phosphate having the function of diluents/bulking agents at the total concentration of 62.75%, carboxymethylcellulose at 3%, which acts as a stabilizer, magnesium stearate at 1.25% and glyceryl dibehenate at 3% with the function of lubricants and, finally, a high concentration of silicon dioxide as an anti-caking agent (Table 25).

*Table 25: base mixture formula 3.*

| Excipient | % w/w |
|---|---|
| Active | 24 |
| Diluents | 62.75 |
| Stabilizer | 3 |
| Lubricants | 4.25 |
| Anti-caking agent | 6 |

As a first step, magnesium stearate was replaced with *glyceryl dibehenate* at two different concentrations, 4.25% and 3% (Table 26).

*Table 26: replacement of magnesium stearate with glyceryl dibehenate.*

| | % w/w | | |
|---|---|---|---|
| **Excipient** | **Base mixture** | **Mixture A** | **Mixture B** |
| **Glyceryl dibehenate** | **3** | **4.25** | **3** |
| Magnesium stearate | 1.25 | - | - |

The data obtained from the flowability tester show no differences between the three mixtures compared, all of them flow only through 25 mm and 15 mm orifices, maintaining a very similar flow time.

A slight difference may be noted in the Hausner and compressibility indices, which assume lower values in mixture B (Table 27). This means that high percentages of *glyceryl dibehenate* do not improve the flow properties, and it is for this reason that all other formulations have been carried out keeping constant the percentage of this ingredient at 3%.

*Table 27: results obtained from the flowability tester and the tapped density tester.*

|  | Flowability | | | H.I | C.I |
|---|---|---|---|---|---|
|  | **25mm** | **15mm** | **10mm** | | |
| Base mixture | yes | yes | no | 1.29 | 22.17 |
| Mixture A | yes | yes | no | 1.27 | 20.95 |
| Mixture B | yes | yes | no | 1.23 | 18.57 |

The rheometer analysis also shows that the flow function of mixture B is lower than that of the base mixture, indicating an improvement in the flow properties (Figure 15). Subsequently, the diluent excipients microcrystalline cellulose and calcium phosphate were replaced with the fructo-oligosaccharides (Table 28).

*Table 28: total and partial replacement with fructo-oligosaccharides.*

| Excipient | % w/w | | |
|---|---|---|---|
|  | **Mixture B** | **Mixture F1** | **Mixture F2** |
| Microcrystalline cellulose | 36.07 | - | 36.07 |
| Calcium phosphate | 26.68 | - | - |
| **Glycerin dibehenate** | **3** | **3** | **3** |
| **Fructo-oligosaccharides** | **.** | **64** | **27.96** |

From the results obtained with the use of the *flowability tester,* it can be seen how the total or partial replacement of microcrystalline cellulose and calcium phosphate is useful in significantly improving the flowability properties; in fact, mixture F1 and mixture F2 flow to all diameters of the instrument, while mixture B does not flow at the diameter of 10 mm.

The Hausner index and the compressibility index are better than the starting mixture, with the new mixtures having a flowability attitude between "good" and "discrete" (table 29).

*Table 29: results obtained from the flowability tester and the tapped density tester.*

|  | Flowability | | | H.I. | C.I. |
|---|---|---|---|---|---|
|  | **25mm** | **15mm** | **10mm** | | |
| Mixture B | yes | yes | no | 1.23 | 18.57 |
| Mixture F1 | yes | yes | yes | 1.16 | 13.81 |
| Mixture F2 | yes | yes | yes | 1.20 | 16.66 |

The mixture F1, due to the presence of high concentrations of fructo-oligosaccharides, during the compression tests caused the phenomenon of descaling, in which the mixture could not be adequately compressed. For this reason, a mixture containing 20% microcrystalline cellulose as a traditional diluent, partially replaced with FOS, was selected for subsequent systematic analyses.

The mixtures prepared until now all presented a visible inhomogeneity with the presence of white lines and spots, due to the high concentration of silicon dioxide. This formula is characterized by two different types of silicon dioxide: 5% Sipernat silicon and 1% Syloid silicon. Both ingredients act as an anti-caking agent, but the first has a granular morphology that facilitates flow and allows moisture to be absorbed. In order to improve the appearance of the mixture and make the surface of the resulting tablets more homogeneous, a formulative systematic analysis was carried out that included different concentrations and different ratios between the two silicon dioxide (Table 30).

*Table 30: systematic with variation of silica dioxide concentrations and ratios.*

| | % w/w | | | | | |
|---|---|---|---|---|---|---|
| Excipient | Mixture S1 | Mixture S2 | Mixture S3 | Mixture S4 | Mixture S5 | Mixture S6 |
| Microcrystalline cellulose | 20 | 20 | 20 | 20 | 20 | 20 |
| Calcium phosphate | - | - | - | - | - | - |
| Glyceryl dibehenate | 3 | 3 | 3 | 3 | 3 | 3 |
| Fructo-oligosaccharides | 50 | 47.5 | 49 | 46.5 | 46 | 46.5 |
| Silicon dioxide Syloid | - | - | 1 | 3.5 | 2 | 1 |
| Silicon dioxide Sipernat | - | 2.5 | - | - | 2 | 1.25 |

The *flowability tester* graphs show that the Syloid silica dioxide ratios: Sipernat equal to 1:1 and 1: 1.25, improve the flowability properties, as the mixtures S5 and S6 flow to all openings of the instrument. Both silica-free mixtures S1 and S2 flow only at 25 mm, while the mixtures S3 and S4 flow at 25 mm and at 15 mm, but not at 10 mm (figure 16). From these results it can be observed that the total replacement of Syloid and Sipernat silicon dioxide with FOS does not improve flowability. **In** fact, the mixture S1, which flows only at 25 mm, has high Hausner and compressibility indexes, presenting an attitude to flowability considered "acceptable". Sipernat silicon dioxide removal is possible, but the mixture S3 does not flow at 10 mm. The increase in Syloid silicon dioxide, mixture S4, leads to inhomogeneity of the mixture; it is therefore advisable to insert this ingredient at a maximum concentration of 1-2%. Finally, the 1:1.25 and 1:1 ratios between Syloid and Sipernat silicon dioxide are the best, since they have an attitude to flowability, given by the Hausner indices and compressibility, "good" and "discreet" (figure 17).

The data obtained at the rheological analysis (Figure 18), confirm the fact that the total replacement of both silicon dioxide or only Syloid silicon dioxide leads to greater *flow functions* than all other mixtures included in the easy flowing range.

For mixtures S3, S5 and S6, the flow functions are located in the free flowing zone and have good flow flowability characteristics.

Finally, a further comparison was made between the starting mixture and the final mixture by introducing, in addition to glyceryl dibehenate and fructo-oligosaccharides, also the excipient arabinogalactan with the function of ancillary diluent (Table 31). A 1:1 silicon dioxide ratio was used, as it was the one that did not exhibit inhomogeneity and did not alter the density and flow properties conferred by the excipients introduced into the mixture.

*Table 31: addition of arabinogalactans*

| | % w/w | | |
|---|---|---|---|
| **Excipient** | **Base mixture** | **Mixture C1** | **Mixture C2** |
| Microcrystalline cellulose | 36.07 | 20 | 20 |
| Calcium phosphate | 26.68 | - | - |
| **Glycerin dibehenate** | **3** | **3** | **3** |
| **Fructo-oligosaccharides** | **-** | **49** | **46.5** |
| **Silicon dioxide Syloid** | **1** | **2** | **2** |
| **Silicon dioxide Sipernat** | **5** | **2** | **2** |
| **Arabinogalactans** | **-** | **-** | **2.5** |

The results obtained at the *flowability tester* show an implementation of the flowability with the introduction of the arabinogalactans, bringing the mixture C2 to flow even at 10 mm (figure 19).

The compressibility index of mixture C2 also shows optimal values, indicating a "discrete/moderate" flowability of the final mixture compared to the base one, which was considered "acceptable" (Figure 20).

The rheological analysis does not report significant differences between the base and C2 mixtures, since the base mixture is already located in the free flowing range, and therefore the replacement of the classic excipients with the innovative ones led to the formulation of a mixture that was in the same flowability area, with better flowability properties at the *flowability tester* and the *tapped density tester,* and with the addition of health properties to the final product.

The complete formulation C2 with the active ingredient Welltan is shown in Table 32 below.

*Table 32 Complete Formulation C2*

| Complete C2 | |
|---|---|
| **Component** | **%** |
| Welltan | 24 |
| Microcrystalline cellulose | 20 |
| FOS | 46.5 |
| Compritol e ato | 3 |
| Silicon SYLOID | 2 |
| Silicon SIPERNAT | 2 |
| Arabinogalactan | 2.5 |

When compared with the corresponding base mixture reported in Table 33

*Table 33 - Base mixture*

| BASE MIXTURE | |
|---|---|
| **Component** | **%** |
| Welltan | 24 |
| Microcrystalline cellulose | 36.07 |

(continued)

| BASE MIXTURE | |
|---|---|
| **Component** | **%** |
| Calcium phosphate | 26.68 |
| Carboxymethylcellulose | 3 |
| Mg stearate | 1.25 |
| Silicon SYLOID | 1 |
| Silicon SIPERNAT | 5 |
| Compritol e ato | 3 |

We observe that with formula C2 46.5% of the base mixture was modified, 61% without active ingredient.

In C2 100% calcium phosphate and 44% microcrystalline cellulose were replaced by FOS. The ratio (FOS + arabinogalactan)/microcrystalline cellulose is : 2.45 :1.

Table 34 shows a new formulation C3 in which the FOS was reduced.

*Table 34*

| C3 | |
|---|---|
| **Component** | **%** |
| Welltan | 24 |
| Microcrystalline cellulose | 36 |
| FOS | 30.5 |
| Compritol e ato | 3 |
| Silicon SYLOID | 2 |
| Silicon SIPERNAT | 2 |
| Galacotomannan | 2.5 |

In C3, 33% of the formula was modified (43.4% without active ingredient).

In C3, 100% calcium phosphate and 100% sodium carboxymethylcellulose was replaced with FOS + Arabinogalactans.

In C3 the weight ratio (FOS+ arabinogalactan) microcrystalline cellulose is 0.91.

Figures 21A, 21B, and 21C show the results of flowability, Hausner index and Flow function of formulations C2 (Test 1) and C3 (Test 2) compared to the base formula. From these data it appears that formula C2 has greater flowability than formulation C3.

*Mixture 4*

The mixture 4 is intended for the formulation of orodispersible tablets that carry an active ingredient that helps to improve the digestion of lactose in people who have difficulty digesting it. For the formulation of mixture 4, analyses were initially conducted to select the best excipient for the formulation of orodispersible tablets.

Table 35 contains the list of excipients that have been analysed with the function of orodispersible disaggregating agents.

*Table 35: orodispersible disaggregating excipients.*

| Initials | Trade name | INCI |
|---|---|---|
| **PF** | PEARLITOL FLASH | Mannitol, Maize starch |

(continued)

| Initials | Trade name | INCI |
|---|---|---|
| PS | PROSOLV ODT G2 | Microcrystalline cellulose, Colloidal silicon dioxide, Mannitol, Fructose, Crospovidone |
| EX | EXCI ODT | Tapioca starch, Erythritol, Bamboo cellulose, Acetylated starch, Silica, Glyceryl dibehenate, Stevia rebaudioside A |
| OM | OMYANUTRA 300 FLASH LEG | Granulated, co-processed, Calcium Carbonate, Tricalcium Phosphate and Cross-linked, Sodium Carboxymethylcellulose (≤3%) |
| SE | SEPISTAB ST 200 | Maize starch, pregelatinised maize starch, sodium sulphite |

The various excipients with the function of disaggregating agents were analysed at the *flowability tester,* the *tapped density tester* and the *rheometer.*

The parameters obtained from the flowability tester highlight the good flow properties of PF and OM, which flow at all openings of the instrument (figure 22).

Finally, PF was selected from these two excipients because it has a Hausner index and a compressibility index that describe a "good" flowability attitude, compared to that of OM considered "discrete", according to the guidelines of the Pharmacopoeia (figure 23).

The rheological analysis also clearly highlights how the excipient PF is the one that has better flowability properties than the other excipients, showing a flow function located in the free flowing range (figure 24).

The base mixture 4 containing 4.5% of active ingredient and the orodispersible excipient selected PF at 81.5% consists of microcrystalline cellulose with the function of diluent/bulking agents, hydroxypropylcellulose acting as a binder, and finally magnesium stearate as an anti-caking agent (Table 36).

*Table 36: formula mixture 4.*

| Excipient | % w/w |
|---|---|
| Active | 4.5 |
| Disaggregating agent ODT | 81.5 |
| Diluent | 10 |
| Binder | 3 |
| Anti-caking agent | 1 |

Initially, the possibility of replacing magnesium stearate with *glyceryl dibehenate* was verified by studying its behaviour within the mixture (Table 37).

*Table 37: replacement of magnesium stearate with glyceryl dibehenate.*

| | % w/w | |
|---|---|---|
| **Excipient** | **Base mixture** | **Mixture A** |
| Magnesium stearate | 1 | - |
| **Glyceryl dibehenate** | **-** | **1** |

The parameters obtained with the *flowability tester* and with the *tapped density tester* (Table 38) show that there are no significant differences between the base mixture and mixture A. This means that the introduction of glyceryl dibehenate in place of magnesium stearate does not compromise the mechanical properties of the mixture.

*Table 38: results obtained at the flowability tester and the tapped density tester.*

| | Flowability | | | H.I | C.I |
|---|---|---|---|---|---|
| | **25mm** | **15mm** | **10mm** | | |
| Base mixture | yes | yes | yes | 1.20 | 16.67 |
| Mixture A | yes | yes | yes | 1.21 | 17.14 |

It is possible to notice a slight difference between the two mixtures in the graph obtained with the rheometer analysis (figure 25), in which the flow function of mixture A is slightly higher, with a small section located in the easy flowing range, compared to the base mixture located entirely within the free flowing zone. In order to optimize the flowability properties by completely replacing magnesium stearate and using glyceryl dibehenate in its place, systematic analyses were formulated that allowed the addition of fructo-oligosaccharides in place of microcrystalline cellulose and hydroxypropylcellulose (table 39).

In mixture F2 a total replacement of microcrystalline cellulose with the fructo-oligosaccharides was carried out, whereas in mixture F2, microcrystalline cellulose was partially replaced and hydroxypropylcellulose was completely replaced.

*Table 39: partial replacement of common excipients with fructo-oligosaccharides.*

| | **% w/w** | | |
|---|---|---|---|
| **Excipient** | **Mixture F1** | **Mixture F2** | **Mixture F3** |
| Microcrystalline cellulose | 10 | - | 5 |
| Disaggregating mannitol (PF) | 81.5 | 81.5 | 81.5 |
| Hydroxypropylcellulose | 3 | 3 | - |
| **Glyceryl dibehenate** | **1** | **1** | **1** |
| **Fructo-oligosaccharides** | **-** | **10** | **8** |

The analysis of the mixtures at the *flowability tester* (Figure 26) showed that, despite all the mixtures, F1, F2 and F3, flow at all three diameters, the times of the mixtures with the fruit-oligosaccharide excipient are still considerably lower than the base mixture. The compressibility index has slightly lower values in the mixture F2 and F3, compared to the mixture F1 in which the fructo-oligosaccharides are not present (Figure 27).

The differences between the mixtures are minimal and this allows us to replace microcrystalline cellulose and hydroxypropylcellulose.

The rheological analysis highlights how the *flow functions* of the mixtures F2 and F3 are fully located in the *"free flowing* range", unlike the mixture F1 which is located in the *"easy flowing"* area (figure 28). This means that the presence of fructo-oligosaccharides in the mixture F2 and F1 improves the flow properties of the mixture.

During the compression tests, mixture F2 exhibited decapping, leading to the formation of non-uniform tablets. Then the mixture F3 was selected as definitive for the addition in the last formula of the ancillary excipient arabinogalactans to verify its behaviour with the other components of the mixture and verify a possible change in the flowability properties (table 40).

*Table 40: addition of arabinogalactans.*

| Excipient | % w/w | |
|---|---|---|
| | Mixture F3 | Mixture C |
| Microcrystalline cellulose | 5 | 5          5 |
| Disaggregating mannitol (PF) | 81.5 | 80 |
| Hydroxypropylcellulose | - | - |
| **Glyceryl dibehenate** | **1** | **1** |
| **Fructo-oligosaccharides** | **8** | **8** |
| **Arabinogalactans** | **-** | **1** |

The complete formulation C, containing the active ingredient, is shown in the following Table 41.

*Table 41 complete formulation C*

| Complete formula C | |
|---|---|
| **Component** | **%** |
| Lactase | 4.5 |
| Microcrystalline cellulose | 5 |
| **FOS** | **8** |
| Mannitol ODT | 80 |
| **Compritol e ato** | **1** |
| **Arabinogalactans** | **1** |

This formula when compared with the base mixture reported in Table 41 was modified for 10.5%, (11% without active ingredient - 67% excluding mannitol).

In this formula 50% microcrystalline cellulose and 100% HPC was replaced by FOS. FOS+arabinogalactan /microcrystalline cellulose ratio 1.8:1.

In figures 29A, 29B and 29C the flowability, Hausner index and flow function of formulation C is compared respectively with those of the base mixture.

From the results shown in these graphs, the final mixture C flows better than the base mixture.

## Claims

1. Tablet obtained by compression of powder mixtures comprising:

    a) At least one active ingredient,
    b) At least one diluent;
    c) At least one lubricating agent
    d) At least one excipient chosen from at least one of the following classes: acidifier, binder, disaggregating agent, anti-caking agent, sweetener, stabiliser

wherein:

i) the diluent b) is a mixture of at least one conventional diluent and fructo-oligosaccharides (FOS),
ii) the lubricating agent is a mono- and/or diglyceride of a fatty acid;
iii) the weight ratio FOS/conventional diluent is between 0.5 and 3, preferably between 0.7 and 2.5

2. Tablet according to Claim 1 further comprising an additional ingredient e) consisting of arabinogalactan, wherein the weight ratio (FOS+ arabinogalactan)/conventional diluent is from 0.7 to 3.5 preferably from 0.9 to 2.7.

3. Tablet according to Claim 1 or 2 wherein the conventional diluent is selected from at least one of the following compounds: isomalt, mannitol, microcrystalline cellulose, calcium phosphate, sorbitol, xylitol, maltitol, isomaltulose, erythritol.

4. Tablet according to any of Claims 1 to 3, wherein the mono and/or diglyceride of the fatty acid is mono and/or diglycerylbehenate or glyceryl dibehenate.

5. Tablet according to any of Claims 1-4, wherein the binder is hydroxypropyl cellulose.

6. Tablet according to any of Claims 1-5, wherein the anti-caking agent is selected from silicon dioxide and calcium carbonate.

7. Tablet according to any of Claims 1-6, wherein the acidifier is selected from tartaric acid, citric acid and ascorbic acid.

8. Tablet according to any of Claims 1-7, wherein the disaggregating agent is chosen from a mixture of cornstarch and mannitol, cross-linked sodium carboxymethylcellulose (CMC), polyvinylpolypyrrolidone (PVPP), starches and derivatives thereof.

9. Tablet according to any of Claims 2-8, in the form of a chewable tablet, wherein the conventional diluent consists of a mixture of mannitol and isomalt and the ratio (FOS+arabinogalactan)/diluent is 1.14 while the mono- and/or diglycerylbehenate is present at concentrations of 2.5 to 3% by weight on total weight of the tablets.

10. Tablet according to any of Claims 2-8 in the form of a swallowable tablet containing as conventional diluent microcrystalline cellulose and possibly hydroxypropylcellulose as binder, wherein the weight ratio (FOS+ arabinogalactan)/conventional diluent is comprised between 1.11 and 2.45 while the content of mono- and/or diglycerylbehenate is comprised between 2.75 and 3% by weight on total weight of the tablet.

11. Tablet according to any of Claims 2-8 in the form of a swallowable tablet containing as conventional diluent microcrystalline cellulose, the anticaking agent consists of a mixture of silicon oxide in amorphous form with d50 of 320 $\mu$m and of silicon in amorphous form, with d50 comprised between 2.0 and 4.5 $\mu$m in weight ratio 1:1, the weight ratio (FOS + arabinogalactan)/conventional diluent is comprised between 0.9 and 2.45, the mono- and/or diglycerylbehenate is present at concentrations of 3% by weight on total weight of the tablet.

12. Tablet according to any of Claims 2-8, in the form of orodispersible tablets, containing as conventional diluent microcrystalline cellulose and as disgregating agent a mixture of cornstarch and mannitol, free of hydroxypropylcellulose as binder, wherein the weight ratio (FOS+arabinogalactan)/conventional diluent is 1.8, the mono- and/or diglycerylbehenate is present at concentrations of 1% by weight on total weight of the tablet.

| Symbol | Name | Definition |
|---|---|---|
| $d_V$ | Volume diameter | Diameter of a sphere with the same volume as the particle |
| $d_s$ | Surface diameter | Diameter of a sphere with the same surface as the particle |
| $d_{st}$ | Stokes diameter | Diameter of a free-falling particle in the laminar flow region (Re<0.2) |
| $d_a$ | Projected area diameter | Diameter of a circle with area equal to the projected area of the particle |
| $d_p$ | Projected area diameter | Diameter of a circle with area equal to the projected area of the particle in casual position |
| $d_c$ | Perimeter diameter | Diameter of a circle with perimeter equal to the projected perimeter of the external line of the particle |
| $d_A$ | Sieve diameter | Minimal width of the opening through which the particle passes |
| $d_F$ | Feret Diameter | Average value of the distance measured between two parallel tangents to the externa line of the particle |
| $d_M$ | Martin Diameter | Average value of the length of a chord separating the particle in two equivalent areas |

Fig.1

| Flowing attitude | Repose Angle (degrees) |
|---|---|
| Excellent | 25-30 |
| Good | 31-35 |
| Discrete (no facilitation needed) | 36-40 |
| Acceptable | 41-45 |
| Poor (requires facilitation by stirring or vibration) | 46-55 |
| Very Poor | 56-65 |
| Extremely poor | >66 |

Fig. 2

| Compressibility Index | Flowing attitude | Hausner index |
|---|---|---|
| 1-10 | Excellent | 1.00-1.11 |
| 11-15 | Good | 1,12-1.18 |
| 16-20 | Discrete | 1.19-1.25 |
| 21-25 | Acceptable | 1.26-1,34 |
| 26-31 | Poor | 1.35-1.45 |
| 32-37 | Very poor | 1.46-1.59 |
| >38 | Extremely poor | >1.60 |

Fig. 3

Fig. 4

Fig.5

Fig.6

Fig. 7

*Fig.8*

EP 4 678 168 A1

## Flowability – Vitamin D3

Fig.9A

## Hausner index – Vitamin D3

Fig.9B

Fig.9C

Fig.10

Fig.11

EP 4 678 168 A1

## Flowability

■ 25 mm   ■ 15 mm   ■ 10 mm

Fig.12

## Compressibility Index

Fig.13

Fig. 14A

Fig.14B

Fig.14 C

Fig.15

EP 4 678 168 A1

Fig.16

Fig.17

Fig.18

Fig.19

Fig.20

## Flowability - Welltan

## Indice di Hausner - Welltan

Figura 21B

Fig.21 C

Fig.22

Fig.23

Fig. 24

Fig. 25

## Flowability

25mm ▪ 15mm ▪ 10mm

Fig. 26

## Compressibility Index

17,14

16,66

14,29

Mixture F1    Mixture F2    Mixture F3

Figura 27

Fig.28

Flowability - Lactase

Fig. 29A

Fig. 29 B

Fig. 29 C

Europäisches
Patentamt

European
Patent Office

Office européen
des brevets

## EUROPEAN SEARCH REPORT

**Application Number**

EP 25 18 8288

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 115 836 701 A (ZHENGZHOU LULUTONG PHARMACEUTICAL TECH CO LTD) 24 March 2023 (2023-03-24) | 1-3,6,8 | INV. A61K9/20 A61K9/00 A61K31/593 A61K31/714 |
| Y | * examples 1-7 * | 1-12 | |
| X | CN 105 851 238 A (WU XINGYANG) 17 August 2016 (2016-08-17) | 1,3,8 | |
| Y | * examples 2-3 * | 1-12 | |
| Y | US 2017/232048 A1 (EDWARDS STEVEN J [US]) 17 August 2017 (2017-08-17) * paragraphs [0094], [0024]; claim 13. 15 * | 1-12 | |
| A | WO 2011/027128 A1 (UNIV MANCHESTER [GB]; PAUS RALF LUDWIG [DE] ET AL.) 10 March 2011 (2011-03-10) * example 8 * | 1-12 | |
| A | SANCHEZ C. ET AL: "The Acacia Gum Arabinogalactan Fraction Is a Thin Oblate Ellipsoid: A New Model Based on Small-Angle Neutron Scattering and Ab Initio Calculation", BIOPHYSICAL JOURNAL, vol. 94, no. 2, 1 January 2008 (2008-01-01), pages 629-639, XP093258861, AMSTERDAM, NL ISSN: 0006-3495, DOI: 10.1529/biophysj.107.109124 * the whole document * | 1-12 | **TECHNICAL FIELDS SEARCHED (IPC)** A61K |

-/--

The present search report has been drawn up for all claims

| Place of search | Date of completion of the search | Examiner |
|---|---|---|
| Berlin | 19 November 2025 | Neumann, Robert |

EPO FORM 1503 03.82 (P04C01)

Europäisches Patentamt
European Patent Office
Office européen des brevets

**EUROPEAN SEARCH REPORT**

**Application Number**

EP 25 18 8288

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DENIS RENARD ET AL: "Acacia s enegal Gum: Continuum of Molecular Species Differing by Their Protein to Sugar Ratio, Molecular Weight, and Charges", BIOMACROMOLECULES, vol. 7, no. 9, 1 September 2006 (2006-09-01), pages 2637-2649, XP055181115, ISSN: 1525-7797, DOI: 10.1021/bm060145j * the whole document *  ----- | 1-12 | |
| | | | **TECHNICAL FIELDS SEARCHED (IPC)** |

The present search report has been drawn up for all claims

| Place of search | Date of completion of the search | Examiner |
|---|---|---|
| Berlin | 19 November 2025 | Neumann, Robert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 18 8288

This annex lists the patent family members relating to the patent documents cited in the above-mentioned European search report.
The members are as contained in the European Patent Office EDP file on
The European Patent Office is in no way liable for these particulars which are merely given for the purpose of information.

19-11-2025

| Patent document cited in search report | | Publication date | Patent family member(s) | | Publication date |
|---|---|---|---|---|---|
| CN 115836701 | A | 24-03-2023 | NONE | | |
| CN 105851238 | A | 17-08-2016 | NONE | | |
| US 2017232048 | A1 | 17-08-2017 | NONE | | |
| WO 2011027128 | A1 | 10-03-2011 | AU | 2010290985 A1 | 19-04-2012 |
| | | | CA | 2772992 A1 | 10-03-2011 |
| | | | CN | 102596205 A | 18-07-2012 |
| | | | EP | 2473175 A1 | 11-07-2012 |
| | | | ES | 2730736 T3 | 12-11-2019 |
| | | | JP | 6086373 B2 | 01-03-2017 |
| | | | JP | 2013503841 A | 04-02-2013 |
| | | | JP | 2016020381 A | 04-02-2016 |
| | | | US | 2012190641 A1 | 26-07-2012 |
| | | | WO | 2011027128 A1 | 10-03-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *What are food supplements? Are they really needed? | Mario Negri.*, 03 April 2024, https://www.marionegri. it/magazine/integratori-alimentari
- Compresse,. **P. COLOMBO** ; **S. NICOLI,**. Principi di Tecnologia Farmaceutica, Casa Editrice Ambrosiana. 2004, 251-268
- Material for Direct Compaction. **G. K. BOLHUIS** ; **Z. T. CHOWHAN** ; **G. ALDERBORN** ; **C. NYSTROM**. Pharmaceutical Compaction Powder Technology. Uppsala, 1995, 419
- **M.-C. MARTINI**. Eccipienti cosmetici o ingredienti tecnologici. *EMC - Cosmetologia Medica e Medicina degli Inestetismi Cutanei*, March 2022, vol. 19 (1), 1-9
- **S. CHAUDHARI P** ; **P. PATIL S**. Pharmaceutical Excipients: A review. *International Journal of Advances in Pharmacy, Biology and Chemistry*, 2012
- **T. KUMAR** ; **S. KUMAR GUPTA** ; **K. PRAJAPATI** ; **D. K. TRIPATHI** ; **V. SHARMA** ; **P. JAIN**. Natural Excipients: A Review. *Asian Journal of Pharmacy and Life Science*, 07 April 2024, vol. 2 (1)
- **C. G. ABRANTES** ; **D. DUARTE** ; **C. P. REIS**. An Overview of Pharmaceutical Excipients: Safe or Not Safe?. *J Pharm Sci*, vol. 105 (7), 2019-2026
- Componenti delle preparazioni farmaceutiche - Eccipienti,. **E. MENEGATTI** ; **A. SCATTURIN** ; **F. BORTOLOTTI** ; **A. DALPIAZ**. Principi di Tecnologia Farmaceutica, Casa Editrice Ambrosiana,. 2004, 155-158
- **R. BETTINI** ; **S. CAGNANI,** ; **M. ARTUSI**. *"Polveri" Casa Editrice Ambrosiana*, 2004, 231-247

- **G. ALDERBOR** ; **R. ROWE C.** ; **R. ROBERTS J.** ; **M. WIKBERG** ; **W. DUNCUN-HEWITT C.** Material properties of importance for powder volume reduction and compact strenght. *Pharmaceutical Powder Compaction Technology*, 1995, 245-375
- Particle Size Analysis. **M. RHODES**. Introduction to Particle Technology. Monash University, 2008, 1-25
- **J. HURLEY**. *SIZING PARTICLES WITH A COULTER COUNTER* (70), 86286-5
- **A. SANTOMASO** ; **P. LAZZARO** ; **P. CANU**. Powder flowability and density ratios: the impact of granules packing. *Chem Eng Sci*, July 2003, vol. 58 (13), 2857-2874
- Powder flow and storage. **R. G. HOLDICH**. Fundamentals of Particle Technology. Midland Information Technology and Publishing
- The use of bulk density measurements as flowability indicators. **E. C. ABDULLAH** ; **D. GELDART**. Powder Technol. March 1999, vol. 102, 151-165
- **M. A. KALEEM** ; **M. Z. ALAM** ; **M. KHAN** ; **S. H. I. JAFFERY** ; **B. RASHID**. An experimental investigation on accuracy of Hausner Ratio and Carr Index of powders in additive manufacturing processes. *Metal Powder Report*, December 2021, vol. 76, S50-S54
- **Y. WANG** ; **S. KOYNOV** ; **B. J. GLASSER** ; **F. J. MUZZIO**. A method to analyze shear cell data of powders measured under different initial consolidation stresses. *Powder Technol*, June 2016, vol. 294, 105-112
- **T. SWIZE** ; **F. OSEI-YEBOAH** ; **M. L. PETERSON** ; **P. BOULAS**. Impact of Shear History on Powder Flow Characterization Using a Ring Shear Tester. *J Pharm Sci*, January 2019, vol. 108 (1), 750-754